# EUROPEAN PATENT APPLICATION

(11) **EP 2 950 100 A1**
(43) Date of publication of application: **02.12.2015**
(21) Application number: 14742913.8
(22) Date of filing: 24.01.2014
(51) Int. Cl.: G01N 33/66, G01N 33/52

(54) **BLOOD GLUCOSE METER**

(30) Priority: 25.01.2013 JP 2013012518
(71) Applicant: Terumo Kabushiki Kaisha, Tokyo 151-0072 (JP)
(72) Inventor: NAGASAWA Yasushi, Ashigarakami-gun Kanagawa 259-0151 (JP); OTSUKI Tomoko, Tokyo 103-0028 (JP); TSUCHIYA Yumiko, Tokyo 103-0028 (JP); SONODA Yuki, Tokyo 103-0028 (JP)
(74) Representative: Casalonga
(86) International application number: PCT/JP2014/051569
(87) International publication number: WO 2014/115850

(57) **Abstract**

A blood glucose meter achieving both high functionality and simplicity is provided, which easily differentiates between measurement of blood glucose level and check of past blood glucose levels by using a minimum number of operation buttons. A cap that protects an optical measuring unit of the blood glucose meter is provided and a micro switch that detects a state in which the cap is attached to or removed from the housing is provided. When an input/output control unit detects that the cap is removed from the housing, the input/output control unit moves from a power saving mode to a measuring mode.

## Description

### Technical Field

The present invention relates to a blood glucose meter that displays a measured blood glucose level on a display screen.

### Background Art

Diabetes where the number of patients rapidly increases is caused by insulin secretion abnormality of pancreas and decrease in insulin sensitivity.

To treat the diabetes, it is important to prevent the onset and progression of complications, and for that purpose, it is inevitable to normally control the blood glucose level. Therefore, a blood glucose measuring device (hereinafter referred to as a blood glucose meter) for a patient or a family member of the patient to easily measure the blood glucose level is developed and the applicant develops a small blood glucose meter for a patient to measure his or her own blood glucose level.

Patent Literature 1 is a patent application related to a blood glucose meter applied by the applicant.

### Citation List

### Patent Literature

Patent Literature 1: JP 2011-64596 A

### Summary of Invention

### Technical Problem

As described above, a diabetic patient has to manage his or her blood glucose level in an appropriate range at all times in daily life. Further a diabetic patient has to go to hospital regularly, report measurement results of the blood glucose level to a doctor, and receive lifestyle guidance. In particular, a doctor recommends a patient who is treated with drugs to measure the blood glucose level a plurality of times a day from a viewpoint that the doctor controls the blood glucose level of the patient in order to prevent complications. Above all, the guideline of IDF (International Diabetes Federation) says that the postprandial blood glucose level of a patient relates to a risk of complications and recommends patients to also measure the postprandial blood glucose level.

In recent years, due to evolution of electronic devices, the blood glucose meter obtains a possibility to be a tool to provide information, which gives a patient motivation to continue managing the blood glucose level, from a mere measuring device.

Therefore, the inventors enhances functions of the blood glucose meter by employing a function to calculate and display an average value of the blood glucose levels of the patient, in particular, a function to display blood glucose level measurement data of the patient, a function to display the transition of blood glucose level of the patient in a graph, and an alarm function to prompt the patient to measure the blood glucose level in order to maintain appropriate blood glucose control of the patient.

On the other hand, the blood glucose meter is used by various patients. Therefore, unlike information devices such as a personal computer, the operation of the blood glucose meter has to be simple. In particular, the blood glucose meter is desired to be able to be operated by a patient unfamiliar with operation of information devices, an elderly patient, and a patient who has vision problems such as loss of visual acuity. However, when various functions are added to the blood glucose meter, the operation of the blood glucose meter may be complicated. Specifically, for example, it is not preferable to indiscriminately increase the number of operation buttons to add functions and to indiscriminately increase the number of layers of menus for calling a desired function.

A new blood glucose meter is required to be configured to be able to provide various content such as a graph display of the blood glucose level and an alarm function to prompt a patient to measure the postprandial blood glucose level without damaging the original purpose of the blood glucose meter, which is to allow anyone to be able to easily measure the blood glucose level.

The present invention is made in view of the above, and an object of the present invention is to provide a blood glucose meter achieving both high functionality and simplicity, which easily differentiates between measurement of blood glucose level and check of past blood glucose levels by using a minimum number of operation buttons.

### Solution to Problem

To solve the above problem, the blood glucose meter of the present invention includes a measuring unit to which a measurement test paper for measuring a blood glucose level of a patient is attached, a cap that covers the measuring unit, a housing which has the measuring unit and to which the cap is attachably and detachably attached, and a switch that detects that the cap is attached to the housing. The blood glucose meter further includes a blood glucose level measuring unit that receives a predetermined signal from the measuring unit and measures the blood glucose level of the patient, a display unit which is provided in the housing and displays the blood glucose level, and a power supply control unit that controls supply of power to the display unit and the blood glucose level measuring unit. When the switch detects that the cap is removed from the housing, an input/output control unit moves to a mode, in which the blood glucose level is measured, by activating the display unit and the blood glucose level measuring unit through the power supply control unit and outputs the measured blood glucose level to the display unit.

### Advantageous Effects of Invention

By the present invention, it is possible to provide a blood glucose meter achieving both high functionality and simplicity, which easily differentiates between measurement of blood glucose level and check of past blood glucose levels by using a minimum number of operation buttons, so that it is possible to contribute to good blood glucose control of the patient.

### Brief Description of Drawings

Figs. 1A to 1C are external views of a blood glucose meter according to an embodiment of the present invention.
Fig. 2 is a block diagram illustrating a hardware configuration of the blood glucose meter.
Fig. 3 is a block diagram illustrating functions of software of the blood glucose meter.
Fig. 4 is a schematic diagram illustrating a field configuration and an example of records of a blood glucose level table.
Fig. 5 is a state transition diagram illustrating a rough state change of the blood glucose meter.
Fig. 6 is a state transition diagram illustrating a change starting from a state in which the blood glucose meter sounds an alarm.
Fig. 7 is a flowchart illustrating a rough operation flow of the blood glucose meter.
Fig. 8 is a flowchart illustrating an operation flow of a content mode of the blood glucose meter.
Fig. 9 is a flowchart illustrating an operation flow of initial screen display processing in the content mode of the blood glucose meter.
Fig. 10 is a flowchart illustrating an operation flow of exception processing in the content mode of the blood glucose meter.
Fig. 11 is a flowchart illustrating an operation flow of memory content display processing in the content mode of the blood glucose meter.
Fig. 12 is a flowchart illustrating an operation flow of memory value display processing in the content mode of the blood glucose meter.
Fig. 13 is a flowchart illustrating an operation flow of average value display processing in the content mode of the blood glucose meter.
Fig. 14 is a schematic diagram illustrating changes of a display screen in the memory content display processing of the content mode.
Fig. 15 is a flowchart illustrating an operation flow of alarm sounding processing of the blood glucose meter.
Figs. 16A and 16B are flowcharts illustrating processing that runs at all times when the blood glucose meter is in any of the content mode, a measuring mode, and a setting mode regardless of the operation mode.
Figs. 17A and 17B are diagrams illustrating a display screen of postprandial alarm.
Fig. 18 is a flowchart illustrating an operation flow of the setting mode of the blood glucose meter.
Fig. 19 is a flowchart illustrating an operation flow of the measuring mode of the blood glucose meter.
Fig. 20 is a flowchart illustrating an operation flow of the measuring mode of the blood glucose meter.
Fig. 21 is a flowchart illustrating an operation flow of exception processing of the measuring mode of the blood glucose meter.
Fig. 22 is a flowchart illustrating an operation flow of blood glucose level recording processing of the measuring mode of the blood glucose meter.
Fig. 23 is a flowchart illustrating an operation flow of button area display processing of the measuring mode of the blood glucose meter.
Fig. 24 is a flowchart illustrating an operation flow of blood glucose level display processing of the content mode and the measuring mode of the blood glucose meter.
Figs. 25A and 25B are display screens displayed on a display unit in the blood glucose level display processing of the measuring mode of the blood glucose meter.
Figs. 26A to 26E are diagrams illustrating a variation of a face mark displayed on the display unit in the blood glucose level display processing of the content mode and the measuring mode of the blood glucose meter.
Figs. 27A to 27D are diagrams illustrating a variation of display screens displayed on the display unit in the blood glucose level display processing of the content mode and the measuring mode of the blood glucose meter.
Figs. 28A and 28B are display screens in the measuring mode and a display screen in the content mode.
Fig. 29 is a table illustrating functions assigned to operation buttons.

### Description of Embodiment

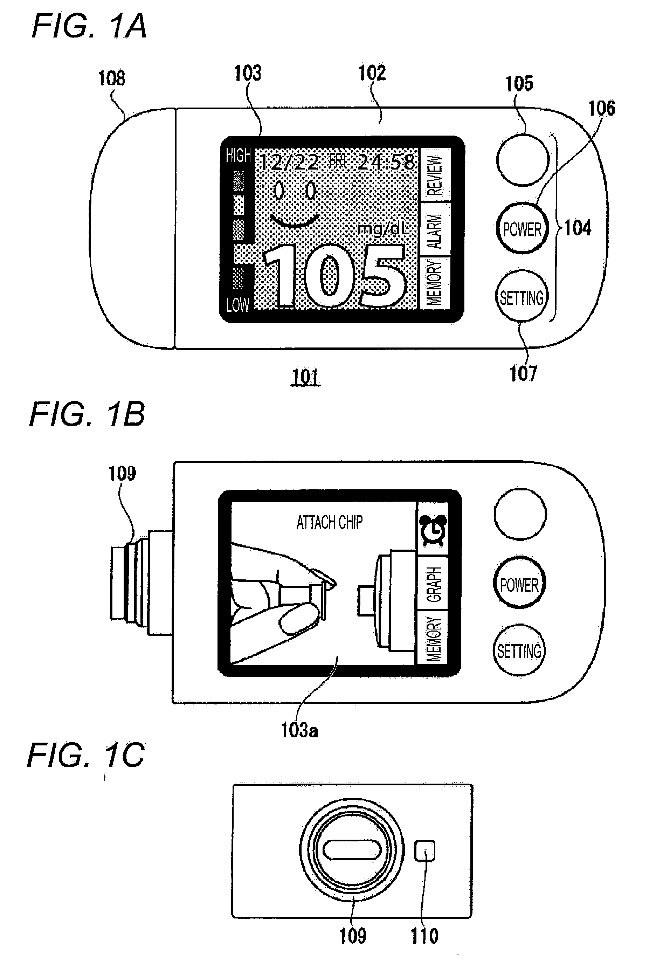

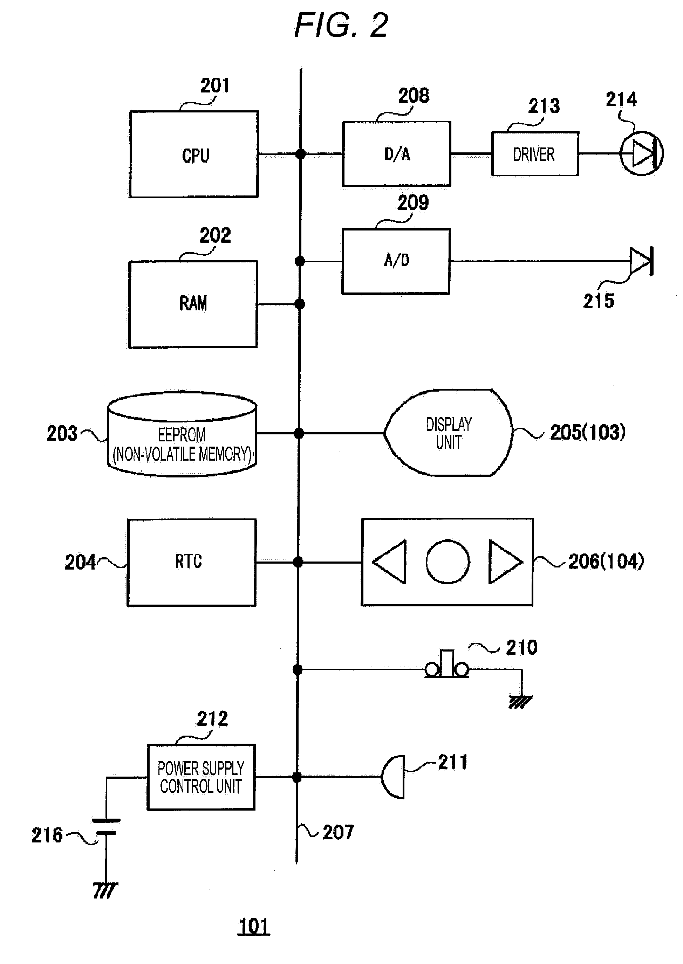

An outline of a blood glucose meter 101 according to the present embodiment will be described with reference to Figs. 1A to 1C and 2.
Figs. 1A, 1B, and 1C are external views of the blood glucose meter 101 according to the embodiment of the present invention.

The blood glucose meter 101 of the present embodiment includes three operation buttons 104 including an upper button 105 that is a first operation button, a middle button 106 that is a second operation button, and a lower button 107 that is a third operation button, and a micro switch 210 (see Fig. 2) interlocked with a cap 108 that protects an optical measuring unit 109.

When the cap 108 is removed from the blood glucose meter 101, the blood glucose meter 101 immediately moves to "measuring mode" in which the blood glucose level is measured.

When the middle button 106 of the blood glucose meter 101 is pressed for less than about one second (hereinafter referred to as "short press"; on the other hand, pressing the operation button 104 for more than about one second is referred to as "long press"), the blood glucose meter 101 moves to "content mode" in which the blood glucose levels that have been measured and stored by the blood glucose meter 101 are displayed and browsed on a liquid crystal display 103. The time used as a threshold value, which differentiates between the short press and the long press, is not limited to one second. For example, the time may be 0.5 seconds.

When the lower button 107 of the blood glucose meter 101 is long pressed, the blood glucose meter 101 moves to "setting mode" in which various setting values of the blood glucose meter 101 are changed.

The blood glucose meter 101 has a so-called postprandial alarm function and clocks a predetermined time when an alarm is set. When the set time has elapsed, the blood glucose meter 101 generates an alarm sound through a buzzer 211 (see Fig. 2) .

The blood glucose meter 101 displays an elapsed time after the alarm sounds on the liquid crystal display 103 for one hour after the alarm sounds.

Further a postprandial flag, which is an example of a prandial flag, is automatically given to a measured blood glucose level for one hour after the alarm sounds.

The prandial flag is a flag that indicates whether the measured blood glucose level is a preprandial blood glucose level or a postprandial blood glucose level by a logical value of the flag. In the present embodiment, the "postprandial flag" which is raised after meal is employed. However, a "preprandial flag" which is raised before meal may be employed.

The upper button 105 of the blood glucose meter 101 of the present embodiment is assigned with a function to switch whether or not to give the postprandial flag during the measuring mode.

Further, the upper button 105 of the blood glucose meter 101 is assigned with a function to set the postprandial alarm during the content mode.

In other words, the upper button 105 has a function related to a concept of "postprandial" in both the measuring mode and the content mode.

The blood glucose meter 101 of the present embodiment has a measurement history display function where the blood glucose levels measured in the past can be retrospectively browsed one by one. Further, the blood glucose meter 101 is provided with an average value display function that displays the oldest blood glucose level and thereafter displays a morning average value, an afternoon average value, and an evening average value of the blood glucose levels measured in the last one month.

Switching between the measurement history display function and the average value display function is seamlessly performed by an operation of the upper button 105 or the lower button 107 without using the menu display.

In the measuring mode, the blood glucose meter 101 of the present embodiment displays a measured blood glucose level in a numerical value and further evaluates the blood glucose level by five levels and displays a face mark according to the evaluation result on a screen of the liquid crystal display 103. Here, the background color of the screen is displayed by being color-coded by five levels. Further, the five levels of color are displayed in a scale form at one end of the screen and a color in the scale matched to the background color is linked to the background color and displayed in a tab shape.

In the measuring mode and the content mode, the blood glucose meter 101 of the present embodiment displays a measured blood glucose level or a past blood glucose level in a numerical value and further evaluates the blood glucose level by five levels and displays a face mark according to the evaluation result on the screen of the liquid crystal display 103. The background color of the screen is displayed by being color-coded by five levels. The presence or absence of the color coding and the face mark can be switched by setting in the setting mode.

In this manner, the blood glucose meter 101 of the present embodiment realizes multiple functions while using a minimum number of operation buttons 104 by assigning different functions to three operation buttons 104 in each of the measuring mode, the content mode, and the setting mode.

### [External View]

With reference to Figs. 1A, 1B, and 1C again, the external view of the blood glucose meter 101 will be described.

The blood glucose meter 101 includes the liquid crystal display 103 that can perform color display on a surface of a housing 102 and the operation buttons 104 formed by known membrane switches and/or touch panel buttons at the right end of the liquid crystal display 103.

The operation buttons 104 are provided outside the liquid crystal display 103 and include the upper button 105, the middle button 106, and the lower button 107.

The upper button 105 is a button to move upward a cursor displayed on the liquid crystal display 103 and the lower button 107 is a button to move the cursor downward. The middle button 106 is a button to specify "execution", "decision", and the like to the blood glucose meter 101.

When the upper button 105 is short pressed in the measuring mode, the upper button 105 functions as a "postprandial button" and records the postprandial flag described later in a corresponding record in a blood glucose level table 306.

When the middle button 106 is short pressed in a power saving mode, the middle button 106 functions as a power button that instructs the blood glucose meter 101 to turn the power on. In the specification of the blood glucose meter 101 of the present embodiment, it is defined that the function to instruct power-on is performed by short press the middle button 106. It maybe defined that the function to instruct power-on is performed by long press the middle button 106.

The lower button 107 has a function to cause the blood glucose meter 101 to enter into the setting mode, in which various setting values of the blood glucose meter 101 are changed, when being long pressed.

The optical measuring unit 109 protected by the removable cap 108 is provided on one short side of the housing 102. A protrusion not illustrated in the drawings is provided inside the cap 108. When the protrusion is engaged with a depression 110 provided in the housing 102, a micro switch 210 (see Fig. 2) included in the housing 102 is turned off.

The blood glucose meter 101 that is driven by a battery not illustrated in the drawings is in a power saving mode in which the blood glucose meter 101 is driven by saved power when the blood glucose meter 101 is in a non-operating state.

When the middle button 106 of the blood glucose meter 101 in the power saving mode is short pressed, the liquid crystal display 103 displays a predetermined menu screen and the blood glucose meter 101 moves to the content mode.

When the cap 108 of the blood glucose meter 101 in the power saving mode is removed and the micro switch 210 is turned on, the liquid crystal display 103 displays a predetermined guide screen and the blood glucose meter 101 moves to the measuring mode.

When the lower button 107 of the blood glucose meter 101 in the power saving mode is long pressed, the liquid crystal display 103 displays a predetermined menu screen and the blood glucose meter 101 moves to the setting mode.

Hereinafter, a state of the power saving mode is referred to as a "power-off state", and states of the content mode, the measuring mode, and the setting mode are collectively referred to as a "power-on state".

As illustrated in Fig. 1B, when the blood glucose meter 101 is moved to the measuring mode by removing the cap 108, an illustration 103a of chip attaching guide is displayed on the liquid crystal display 103 to immediately measure the blood glucose level.

### [Hardware]

Fig. 2 is a block diagram illustrating a hardware configuration of the blood glucose meter 101.

The blood glucose meter 101 is formed by a known microcomputer in which a known CPU 201, RAM 202, EEPROM 203, a real-time clock (hereinafter referred to as "RTC") 204 which is a timing unit that outputs date and time information, a display unit 205 which is the liquid crystal display 103, and an operation unit 206 which includes the operation buttons 104 are connected to a bus 207.

Further, a D/A converter 208, anA/D converter 209, a micro switch 210, a buzzer 211, and a power supply control unit 212 are connected to the bus 207.

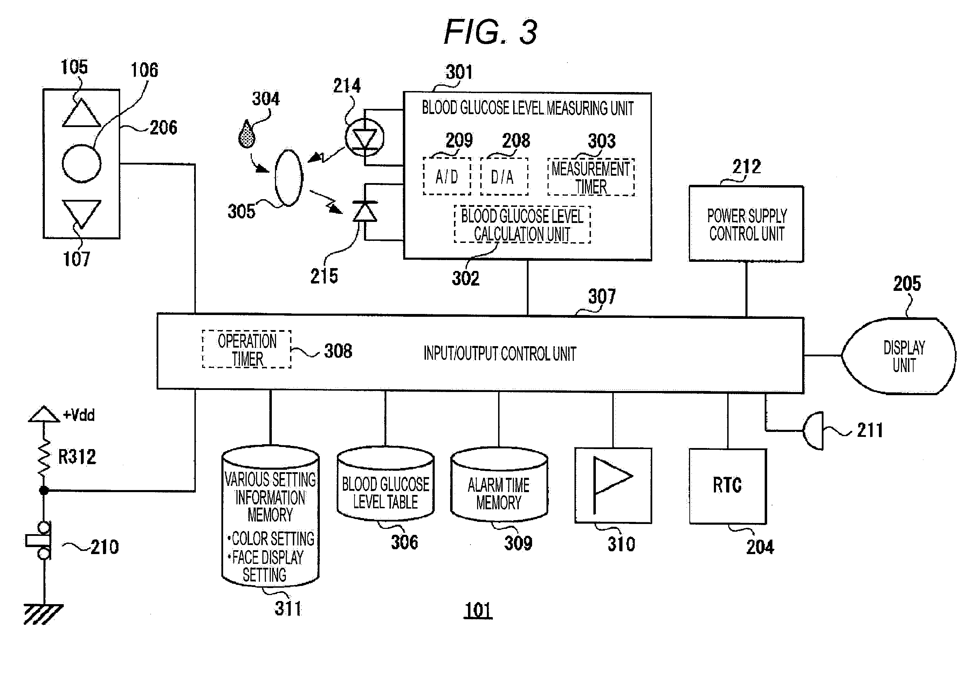

A driver 213 is connected to the D/A converter 208, and further, an LED 214 is connected to the driver 213. The LED 214 is required to irradiate a test paper 305 (see Fig. 3) in a measurement chip with light of appropriate intensity, so that the LED 214 is controlled to emit light based on intensity data stored in the EEPROM 203 described later.

A photodiode 215 is connected to the A/D converter 209.

The photodiode 215 receives the light of the LED 214 reflected by the test paper 305 (see Fig. 3) and generates an electric current according to the intensity of the light. The electric current is converted into numerical data by the A/D converter 209. The numerical data is stored in predetermined areas in the RAM 202 and the EEPROM 203 through predetermined calculation processing.

The LED 214 and the photodiode 215 are housed inside the optical measuring unit 109.

The RTC 204 is an IC that provides a known date and time data output function.

The blood glucose meter 101 of the present embodiment records the date and time information of the time when the blood glucose level is measured in the blood glucose level table 306 (see Fig. 3) along with the blood glucose level.

Various screens are displayed on the display unit 205 by a program which is stored in the EEPROM 203 and executed by the CPU 201. The details of the display screen will be described later.

The buzzer 211 is used to notify an operator of mainly a completion of measurement of the blood glucose level measurement, a notification sound of an alarm described later, or an error message.

The power supply control unit 212 connected to a battery 216 controls the entire power supply of the blood glucose meter 101. The blood glucose meter 101 includes the RTC 204, so that the RTC 204 is required to run at all times regardless of the power-on state and the power-off state. Even in the power-off state, that is, the power saving mode, the blood glucose meter 101 is required to detect a state change of the operation unit 206 and the micro switch 210. Therefore, in the power saving mode, the power supply control unit 212 stops supplying power to devices that have to run only in the power-on state, such as the display unit 205, the D/A converter 208, the driver 213, and the A/D converter 209. Further, in the power saving mode, the power supply control unit 212 lowers the oscillation frequency of a system clock (not illustrated in the drawings) that causes the CPU 201 to run.

### [Functional Configuration]

Fig. 3 is a block diagram illustrating functions of software of the blood glucose meter 101.

As described above, the blood glucose meter 101 is an electronic device formed with a known microcomputer at its center. The microcomputer reads a program (not illustrated in the drawings) stored in the EEPROM 203 and forms functional blocks illustrated in Fig. 3.

A blood glucose level measuring unit 301 measures a blood glucose level by detecting a change of optical reflectivity of the test paper 305 whose color is changed by blood 304 of a measurement object person. The blood glucose level measuring unit 301 includes the D/A converter 208, the A/D converter 209, a blood glucose level calculation unit 302, and a measurement timer 303.

A basic mechanism of the blood glucose meter 101 to measure the blood glucose level is the same as that of conventional techniques. Hereinafter, the mechanism to measure the blood glucose level will be briefly described.

A measuring chip (not illustrated in the drawings) is attached to the optical measuring unit 109, and the blood 304 of the measurement object person is suctioned by the measuring chip. The test paper 305 formed of a porous film such as polyether sulfone is included in the measuring chip. When the blood 304 suctioned by the measuring chip reaches the test paper 305, the blood 304 reacts with a reagent contained in the test paper 305 and generates a color. Subsequently, the light emitted from the LED 214, which is a light-emitting element, is irradiated to the test paper 305, and the light reflected from the test paper 305 is received by the photodiode 215, which is a light-receiving element. After a predetermined reaction time has elapsed, an analog receiving light intensity signal obtained from the photodiode 215 is converted into a digital value by the A/D converter 209, and thereafter the digital value is converted into a blood glucose level.

The mechanism to measure the blood glucose level is not limited to the aforementioned optical measurement method using a color-generating reagent, but it is possible to employ mechanisms that can be conventionally used to measure the blood glucose level, such as an electrochemical sensor method.

The measurement timer 303 is a timer to time a predetermined reaction time from when the blood 304 is suctioned by the test paper 305. In the case of the blood glucose meter 101 of the present embodiment, as an example, the measurement timer 303 times 9 seconds.

When the measurement timer 303 times the reaction time, the blood glucose level calculation unit 302 calculates the blood glucose level by performing predetermined calculation processing based on data indicating the intensity of the reflected light obtained from the photodiode 215 through the A/D converter 209.

The RTC 204 outputs date and time information. The RTC 204 is supplied with power even in the power-off state (the power saving mode) from a battery not illustrated in the drawings, so that the RTC 204 outputs correct date and time information.

The blood glucose level table 306 is a table in which a measurement date and time, a measured blood glucose level, and a postprandial flag described later are recorded. The blood glucose level table 306 is provided in non-volatile memory such as the EEPROM 203.

The operation unit 206 is the aforementioned operation buttons 104. The display unit 205 is the aforementioned liquid crystal display 103.

An input/output control unit 307 additionally records blood glucose data obtained from the blood glucose level measuring unit 301 and a measurement date and time obtained from the RTC 204 in the blood glucose level table 306. Further, the input/output control unit 307 displays the measured blood glucose level on the display unit 205 and performs various data processing according to an operation of the operation unit 206.

The micro switch 210 is also connected to the input/output control unit 307. One end of the micro switch 210 is pulled up by a resistor R312 . The input/output control unit 307 detects whether the cap 108 is attached to the housing 102 of the blood glucose meter 101 or removed from the housing 102 by detecting a change of potential of the micro switch 210.

Further, an operation timer 308 is provided in the input/output control unit 307. The operation timer 308 is reset by a state change of the operation unit 206, the micro switch 210, and the blood glucose level measuring unit 301. For example, the operation timer 308 times up to two minutes. When the input/output control unit 307 detects that the operation timer 308 times two minutes, the input/output control unit 307 determines that a time out occurs and moves to the power saving mode.

In a state in which a menu screen of the content mode is displayed, when the upper button 105 is short pressed, the input/output control unit 307 acquires current date and time information from the RTC 204 and sounds a buzzer 211 (sounds an alarm) in two hours. In this case, the input/output control unit 307 stores the time when the alarm is sounded in an alarm time memory 309. The alarm time memory 309 is required to continuously store the alarm sounding time even in the power saving mode, so that the alarm time memory 309 is provided in the EEPROM 203 which is non-volatile memory.

A postprandial flag memory 310 is, for example, a memory area which includes one-bit variables, which is provided in the EEPROM 203 that is non-volatile memory, and which stores a state of the postprandial flag. The state of the postprandial flag stored in the postprandial flag memory 310 is changed by an operation of the upper button 105 in the measuring mode or is changed between a time period within one hour after the alarm sounds and the other time period.

A various setting information memory 311 is a memory area which is provided in the EEPROM 203 that is non-volatile memory and which stores various setting information of the blood glucose meter 101. The various setting information memory 311 includes a color setting and a face display setting of the display unit 205.

The alarm time memory 309, the postprandial flag memory 310, and the various setting information memory 311 may be provided in the RAM 202 (Battery-Backed Memory) whose storage content is backed up by a battery.

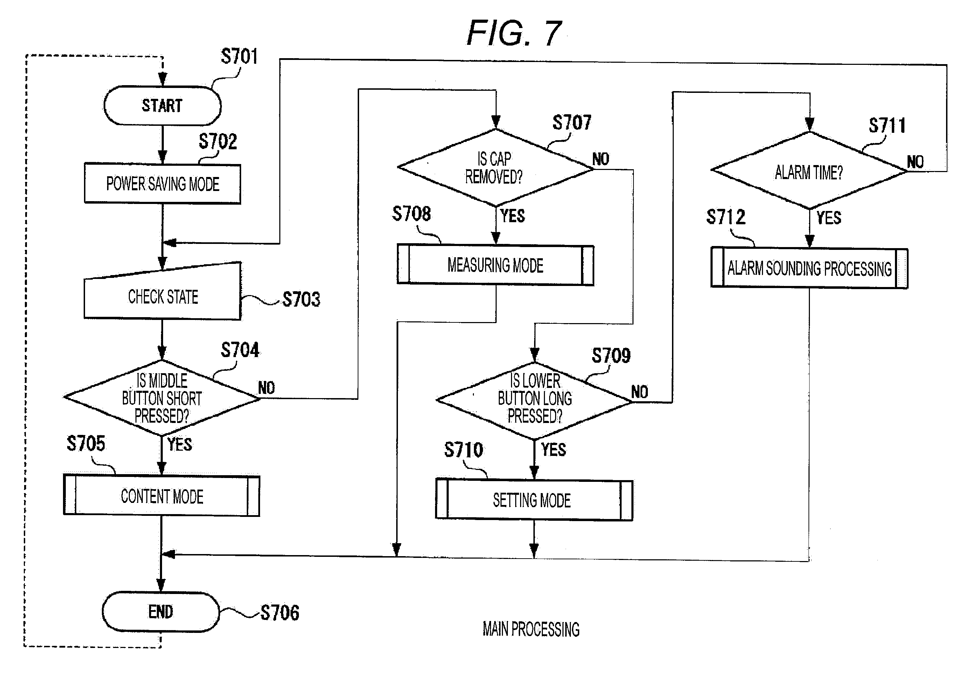

The input/output control unit 307 detects a state change of the operation unit 206 and the micro switch 210 and realizes functions according to the state change. The functions will be described in detail based on the flowcharts illustrated in Fig. 7 and the following drawings.

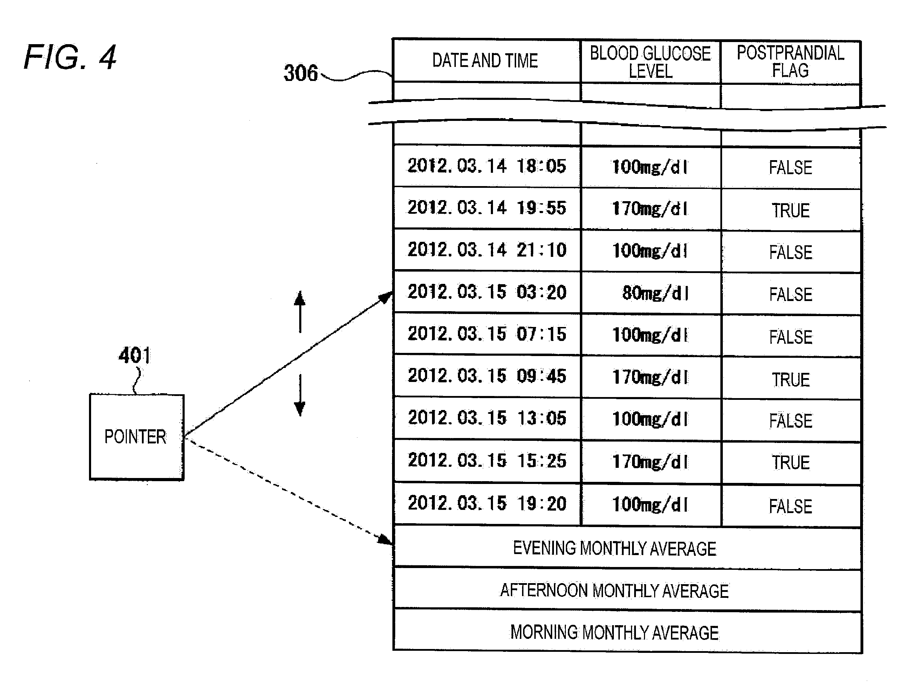

Fig. 4 is a schematic diagram illustrating a field configuration and an example of records of the blood glucose level table 306.

A date and time field stores date and time information of the time when the blood glucose level is measured.

A blood glucose level field stores the measured blood glucose level.

When measuring the blood glucose level, by short press the upper button 105, the postprandial flag indicating that the blood glucose level to be measured is a postprandial blood glucose level is stored in a postprandial flag field.

In the blood glucose level table 306, records in which the blood glucose level is recorded are recorded in chronological order. A label area which is different from the records of the blood glucose level table 306 is provided at the end of the blood glucose level table 306. In the label area, information indicating "evening monthly average", "afternoon monthly average", and "morning monthly average" is stored.

When a memory value display function is performed in the content mode described later, a record of the blood glucose level table 306, which is indicated by a pointer 401 provided in the RAM 202, is displayed on the display unit 205 and when the pointer 401 indicates the label area, a monthly average of the blood glucose levels is displayed on the display unit 205.

### [State Transition]

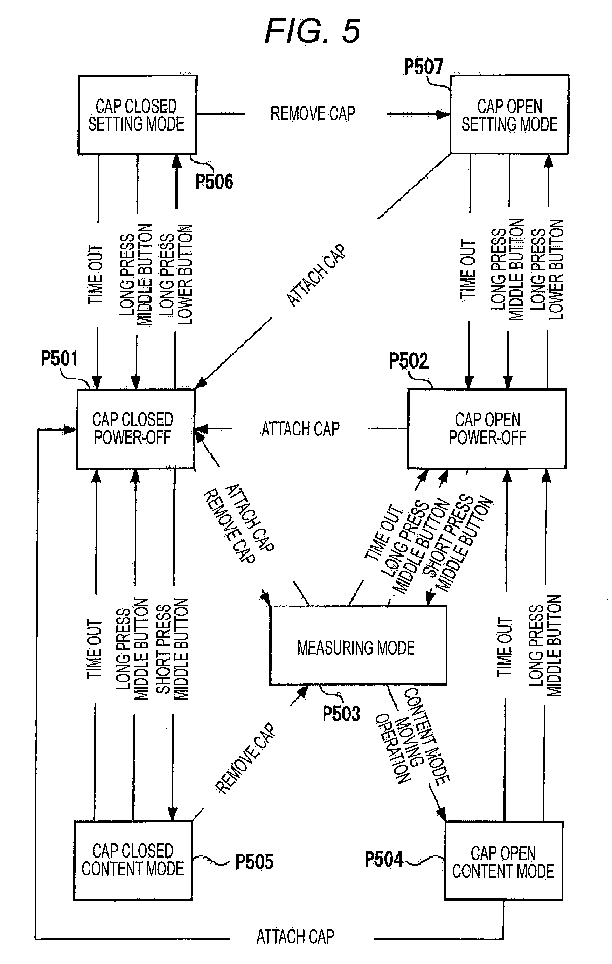

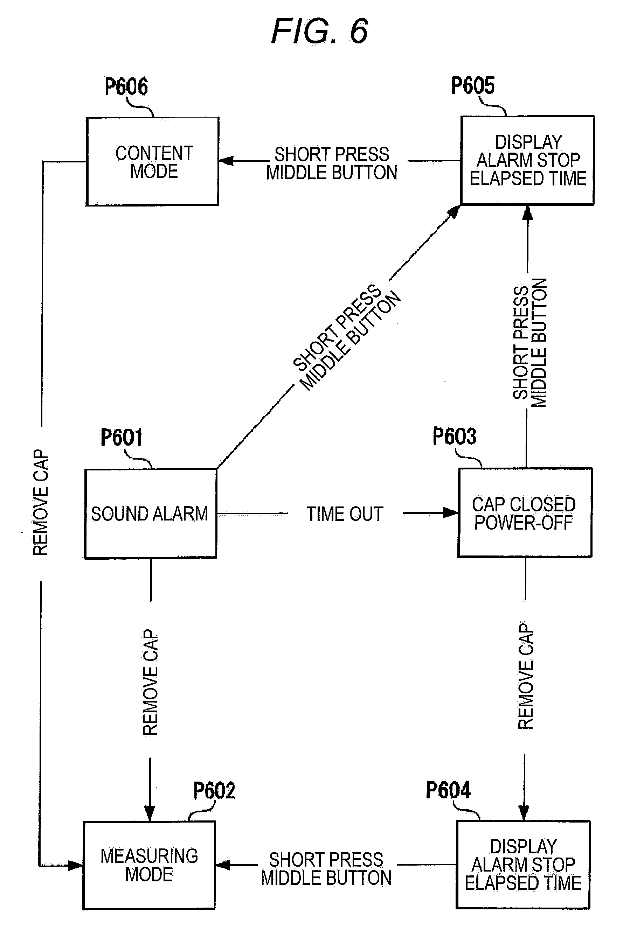

Next, a rough state change of the blood glucose meter 101 will be described with reference to state transition diagrams in Figs. 5 and 6.

First, a rough state change of the blood glucose meter 101 will be described with reference to Fig. 5.

Fig. 5 is a state transition diagram illustrating a rough state change of the blood glucose meter 101.

The initial state of the blood glucose meter 101 is the power saving mode. There are two types of power saving modes: one is a cap 108 closed power-off (P501) which is a power saving mode in a state in which the cap 108 is attached and the other is a cap 108 open power-off (P502) which is a power saving mode in a state in which the cap 108 is removed.

When the cap 108 is removed in a state of the cap 108 closed power-off (P501), the blood glucose meter 101 moves to the measuring mode (P503).

When the cap 108 is attached in a state of the measuring mode (P503), the blood glucose meter 101 moves to the cap 108 closed power-off (P501).

In a state of the measuring mode (P503), when no operation is performed for a predetermined period of time (for example, two minutes) and time out occurs or the middle button 106 is long pressed, the blood glucose meter 101 moves to the cap 108 open power-off (P502).

When the middle button 106 is short pressed in a state of the cap 108 open power-off (P502), the blood glucose meter 101 moves to the measuring mode (P503).

When the middle button 106 is short pressed in a state of the cap 108 closed power-off (P501), the blood glucose meter 101 moves to a cap closed content mode (P505).

When the cap 108 is removed in a state of the cap closed content mode (P505), the blood glucose meter 101 moves to the measuring mode (P503).

In a state of the cap closed content mode (P505), when no operation is performed for a predetermined period of time and time out occurs or the middle button 106 is long pressed, the blood glucose meter 101 moves to the cap 108 closed power-off (P501).

In a state of the measuring mode (P503), when an operation of the operation button 104 tomove to the content mode is performed, the blood glucose meter 101 moves to a cap open content mode (P504).

When the cap 108 is attached in a state of the cap open content mode (P504), the blood glucose meter 101 moves to the cap 108 closed power-off (P501).

In a state of the cap open content mode (P504), when no operation is performed for a predetermined period of time and time out occurs or the middle button 106 is long pressed, the blood glucose meter 101 moves to the cap 108 openpower-off (P502).

When the lower button 107 is long pressed in a state of the cap 108 closed power-off (P501), the blood glucose meter 101 moves to a cap closed setting mode (P506).

In a state of the cap closed setting mode (P506), when no operation is performed for a predetermined period of time and time out occurs or the middle button 106 is long pressed, the blood glucose meter 101 moves to the cap 108 closed power-off (P501).

When the cap 108 is removed in a state of the cap closed setting mode (P506), the blood glucose meter 101 moves to a cap open setting mode (P507).

When the lower button 107 is long pressed in a state of the cap 108 open power-off (P502), the blood glucose meter 101 moves to the cap open setting mode (P507).

In a state of the cap open setting mode (P507), when no operation is performed for a predetermined period of time and time out occurs or the middle button 106 is long pressed, the blood glucose meter 101 moves to the cap 108 openpower-off (P502) .

When the cap 108 is attached in a state of the cap open setting mode (P507), the blood glucose meter 101 moves to the cap 108 closed power-off (P501).

In a state of the cap 108 closed power-off (P501) and a state of the cap 1 closed content mode (P505), when an operation of "removing the cap 108" is performed, the blood glucose meter 101 moves to the measuring mode (P503). In other words, the operation of removing the cap 108 is an instruction to immediately turn on power and at the same time is an instruction to start measurement of the blood glucose level.

In a state of the measuring mode (P503), a state of the cap open content mode (P504), and a state of the cap open setting mode (P507), when an operation of "attaching the cap 108" is performed, the blood glucose meter 101 moves to the cap 108 closed power-off (P501). In other words, the operation of attaching the cap 108 is an instruction to immediately turn off power.

Fig. 6 is a state transition diagram illustrating a change starting from a state in which the blood glucose meter 101 sounds an alarm. For simplicity of the description, when the alarm sounds, the cap 108 is assumed to be attached.

When the cap 108 is removed in a state of alarm sounding (P601), the blood glucose meter 101 moves to a measuring mode (P602).

In a state of the alarm sounding (P601), when no operation is performed for a predetermined period of time and time out occurs, the blood glucose meter 101 moves to a cap 108 closed power-off (P603).

When the cap 108 is removed in a state of the cap 108 closed power-off (P603), the blood glucose meter 101 moves to an alarm stop elapsed time display (P604) in which an elapsed time from when the alarm stops is displayed on the screen.

When the middle button 106 is short pressed in a state of the alarm stop elapsed time display (P604), the blood glucose meter 101 moves to the measuring mode (P602).

When the middle button 106 is short pressed in a state of the alarm sounding (P601), the blood glucose meter 101 moves to an alarm stop elapsed time display (P605).

When the middle button 106 is short pressed in a state of the cap 108 closed power-off (P603), the blood glucose meter 101 moves to the alarm stop elapsed time display (P605).

When the middle button 106 is short pressed in a state of the alarm stop elapsed time display (P605), the blood glucose meter 101 moves to a content mode (P606).

When the cap 108 is removed in a state of the content mode (P606), the blood glucose meter 101 moves to the measuring mode (P602).

One of the features of the blood glucose meter 101 is the postprandial alarm. A period of time when the postprandial blood glucose level can be measured is limited, so that an alarm is set in advance at a time that is assumed to be postprandial. The postprandial alarm sounds an alarm when the set time comes and displays an elapsed time on the liquid crystal display 103. The elapsed time is displayed on the liquid crystal display 103, so that a patient can determine whether or not it is an appropriate time to measure the postprandial blood glucose level.

The postprandial alarm sets the postprandial flag to logical "true" at the same time as sounding an alarm, so that it is not necessary to operate the upper button 105 to give the postprandial flag when measuring the blood glucose level.

### [Flow of Operation]

Fig. 7 is a flowchart illustrating a rough operation flow of the blood glucose meter 101.

When the processing starts by attaching a battery or the like (S701), the input/output control unit 307 first controls the power supply control unit 212 and sets an operational state of the blood glucose meter 101 to the power saving mode (S702).

Subsequently, the input/output control unit 307 checks states of the operation buttons 104, the micro switch 210, and the RTC 204 in a state of the power saving mode (S703).

If the middle button 106 is short pressed (YES in S704), the input/output control unit 307 sets the operational state of the blood glucose meter 101 to the content mode and performs processing of the content mode (S705). When the processing of the content mode ends, the input/output control unit 307 ends a series of processing steps (S706). Then, the processing is started from step S701 again, and the blood glucose meter 101 sets the operational state to the power saving mode in step S702.

In step S704, if the middle button 106 is not short pressed (NO in S704), the input/output control unit 307 checks the state of the micro switch 210. As a result, if the cap 108 is removed (YES in S707), the input/output control unit 307 sets the operational state of the blood glucose meter 101 to the measuring mode and performs processing of the measuring mode (S708). When the processing of the measuring mode ends, the input/output control unit 307 ends a series of processing steps (S706). Then, the processing is started from step S701 again, and the blood glucose meter 101 sets the operational state to the power saving mode in step S702.

In step S707, if the cap 108 is not removed (NO in S707), the input/output control unit 307 checks the state of the lower button 107. As a result, if the lower button 107 is long pressed (YES in S709), the input/output control unit 307 sets the operational state of the blood glucose meter 101 to the setting mode and performs processing of the setting mode (S710). When the processing of the setting mode ends, the input/output control unit 307 ends a series of processing steps (S706). Then, the processing is started from step S701 again, and the blood glucose meter 101 sets the operational state to the power saving mode in step S702.

In step S709, if the lower button 107 is not long pressed (NO in S709), the input/output control unit 307 checks the state of the RTC 204. As a result, if the current time reaches an alarm setting time (YES in S711), the input/output control unit 307 performs alarm sounding processing (S712). When the alarm sounding processing ends, the input/output control unit 307 ends a series of processing steps (S706). Then, the processing is started from step S701 again, and the blood glucose meter 101 sets the operational state to the power saving mode in step S702.

In step S711, if the current time does not reach the alarm setting time (NO in S711), the input/output control unit 307 repeats the processing from step S703 again and checks the states of the operation buttons 104, the micro switch 210, and the RTC 204 in a state of the power saving mode (S703).

### [Content Mode]

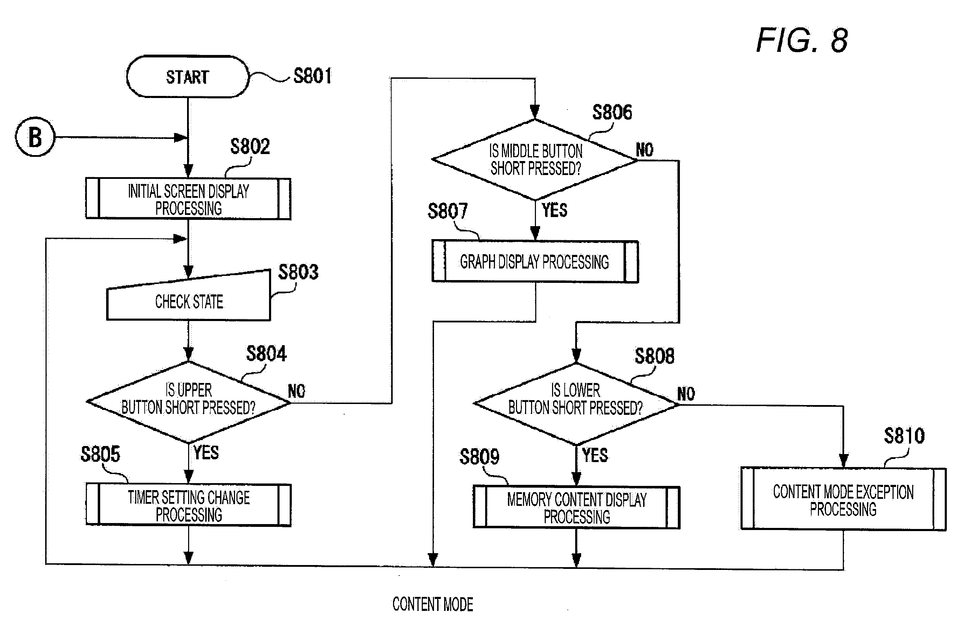

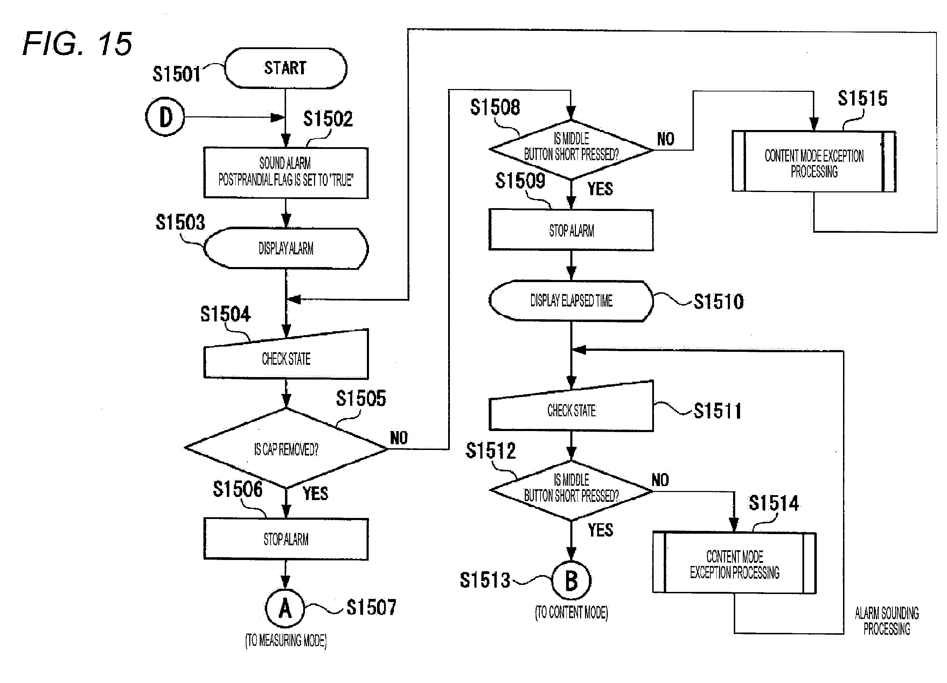

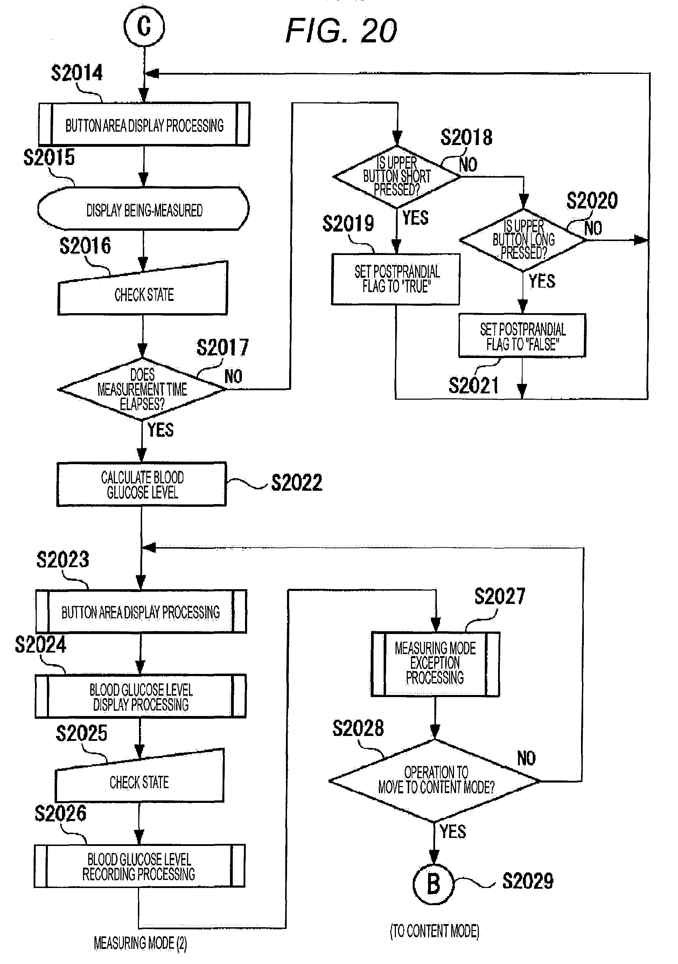

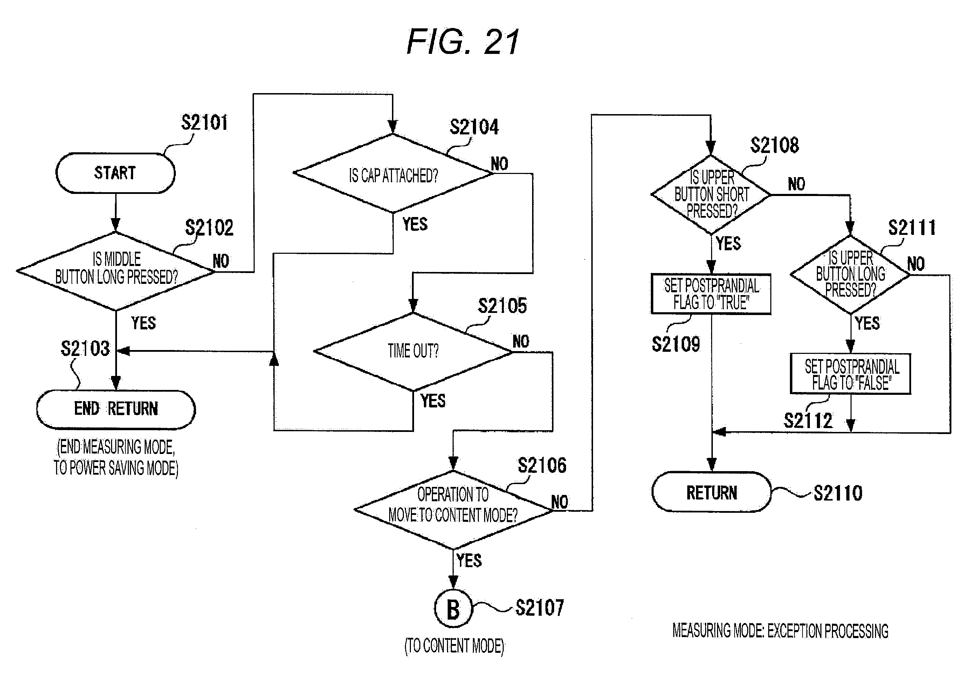

Fig. 8 is a flowchart illustrating an operation flow of the content mode of the blood glucose meter 101. The content mode corresponds to step S705 in Fig. 7 and is performed from step S1513 in Fig. 15, step S2029 in Fig. 20, and step S2107 in Fig. 21, which will be described later.

When the processing of step S705 in Fig. 7 is started (S801) or when the processing of step S1513 in Fig. 15, step S2029 in Fig. 20, or step S2107 in Fig. 21 is started (label "B"), the input/output control unit 307 first performs initial screen display processing (S802).

Subsequently, the input/output control unit 307 checks the states of the operation buttons 104 and the micro switch 210 (S803). As a result, if the upper button 105 is short pressed (YES in S804), the input/output control unit 307 performs timer setting change processing (S805). When the timer setting change processing ends, the input/output control unit 307 checks again the states of the operation buttons 104 and the micro switch 210 (S803).

In step S804, if the upper button 105 is not short pressed (NO in S804), the input/output control unit 307 checks the state of the middle button 106. As a result, if the middle button 106 is short pressed (YES in S806), the input/output control unit 307 performs graph display processing (S807). When the graph display processing ends, the input/output control unit 307 checks again the states of the operation buttons 104 and the micro switch 210 (S803).

In step S806, if the middle button 106 is not short pressed (NO in S806), the input/output control unit 307 checks the state of the lower button 107. As a result, if the lower button 107 is short pressed (YES in S808), the input/output control unit 307 performs memory content display processing (S809). When the memory content display processing ends, the input/output control unit 307 checks again the states of the operation buttons 104 and the micro switch 210 (S803).

In step S808, if the lower button 107 is not short pressed (NO in S806), the input/output control unit 307 performs content mode exception processing (S809). When the content mode exception processing ends, the input/output control unit 307 checks again the states of the operation buttons 104 and the micro switch 210 (S803).

### [Content Mode: Initial Screen Display Processing]

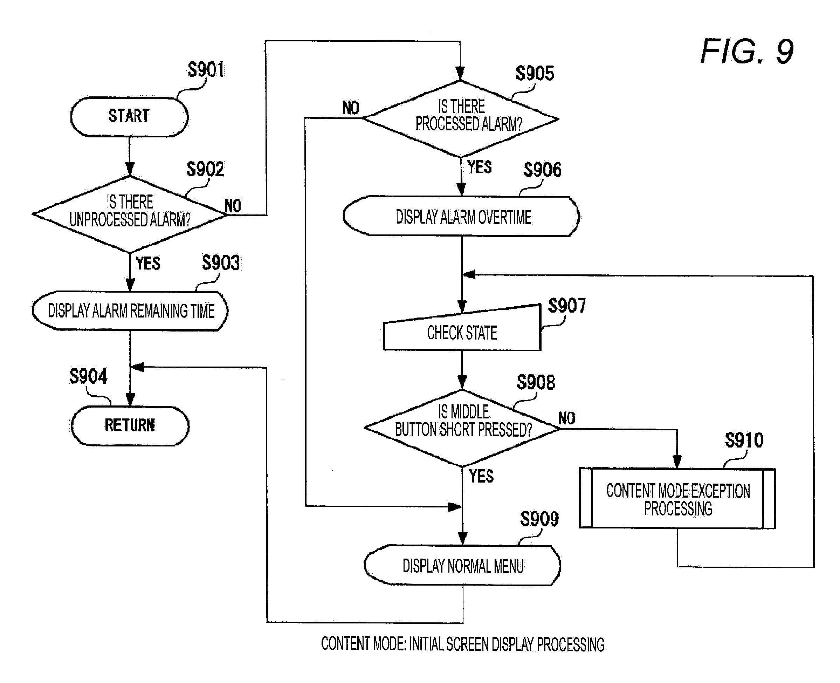

Fig. 9 is a flowchart illustrating an operation flow of the initial screen display processing in the content mode of the blood glucose meter 101. Fig. 9 corresponds to step S802 in Fig. 8.

When the processing starts (S901), the input/output control unit 307 first obtains current date and time information from the RTC 204 and determines whether or not it is within two hours before the alarm sounding time, that is, whether or not the postprandial alarm has not been sounded, by seeing the alarm sounding time stored in the alarm time memory 309 (S902). If it is within two hours before the alarm sounding time (YES in S902), the input/output control unit 307 displays a remaining time of the postprandial alarm on the display unit 205 (S903) and ends a series of processing steps (S904).

In step S902, if it is not within two hours before the alarm sounding time (NO in S902), the input/output control unit 307 determines whether or not it is within one hour after the alarm sounding time, that is, whether or not one hour has not elapsed after the postprandial alarm sounded (S905). If it is within one hour after the alarm sounding time (YES in S905), the input/output control unit 307 displays an alarm overtime of the postprandial alarm on the display unit 205 (S906), and thereafter the input/output control unit 307 checks the states of the operation buttons 104 and the micro switch 210 (S907). As a result, if the middle button 106 is short pressed (YES in S908), the input/output control unit 307 displays a normal menu screen of the content mode on the display unit 205 (S909) and ends a series of processing steps (S904).

In step S908, if the middle button 106 is not short pressed (NO in S908), the input/output control unit 307 performs exception processing of the content mode (S910) and checks again the states of the operation buttons 104 and the micro switch 210 (S907).

### [Content Mode: Exception Processing]

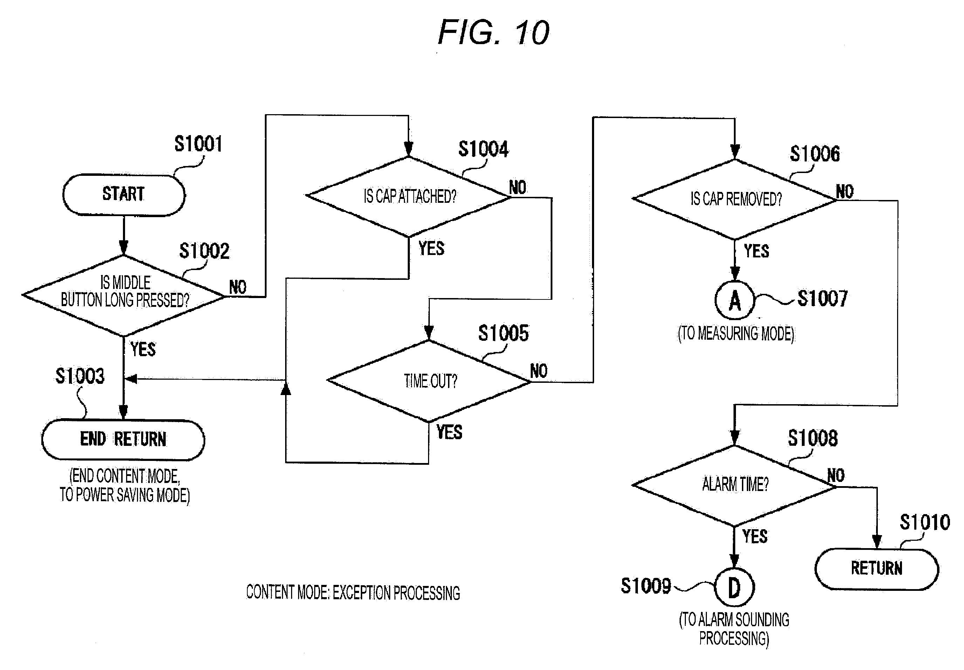

Fig. 10 is a flowchart illustrating an operation flow of the exception processing in the content mode of the blood glucose meter 101. Fig. 10 corresponds to step S810 in Fig. 8 and step S910 in Fig. 9.

When the processing starts (S1001), the input/output control unit 307 determines whether or not the middle button 106 is long pressed (S1002). If the middle button 106 is long pressed (YES in S1002), the input/output control unit 307 ends the processing of the content mode (S1003). Thereby, the blood glucose meter 101 moves to the power saving mode (step S702 in Fig. 7).

In step S1002, if the middle button 106 is not long pressed (NO in S1002), the input/output control unit 307 sees the state of the micro switch 210 and determines whether or not the cap 108 is attached (S1004). If the cap 108 is attached (YES in S1004), the input/output control unit 307 ends the processing of the content mode (S1003). Thereby, the blood glucose meter 101 moves to the power saving mode (step S702 in Fig. 7).

In step S1004, if the cap 108 is not attached (NO in S1004), the input/output control unit 307 sees the operation timer 308 and determines whether or not the operation timer 308 reaches timeout (S1005). If the operation timer 308 reaches timeout (YES in S1005), the input/output control unit 307 ends the processing of the content mode (S1003). Thereby, the blood glucose meter 101 moves to the power saving mode (step S702 in Fig. 7).

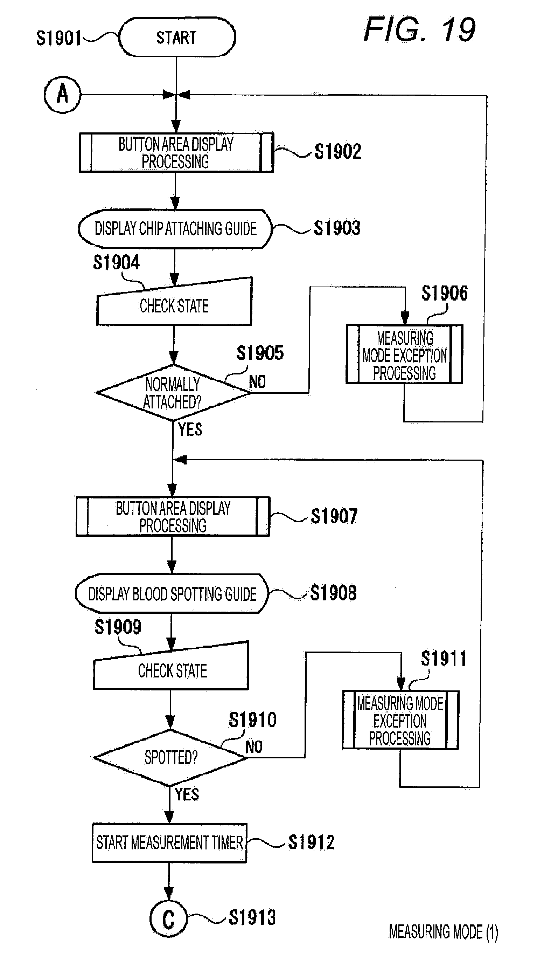

In step S1005, if the operation timer 308 does not reach timeout (NO in S1005), the input/output control unit 307 sees the state of the micro switch 210 and determines whether or not the cap 108 is removed (S1006). If the cap 108 is removed (YES in S1006), the input/output control unit 307 moves the processing from the content mode to the measuring mode (S1007). Thereby, the blood glucose meter 101 moves to the measuring mode (from S708 in Fig. 7 to label "A" in Fig. 19; details will be described later with reference to Fig. 19).

In step S1006, if the cap 108 is not removed (NO in S1006), the input/output control unit 307 obtains the current date and time information from the RTC 204 and at the same time sees the alarm sounding time stored in the alarm time memory 309 and determines whether or not the current time reaches the alarm sounding time (S1008).

If the current time reaches the alarm time (YES in S1008), the input/output control unit 307 moves the processing from the content mode to the alarm sounding mode (S1009). Thereby, the blood glucose meter 101 moves to the alarm sounding mode (from S712 in Fig. 7 to label "D" in Fig. 15; details will be described later with reference to Fig. 15).

In step S1008, if the current time does not reach the alarm time (NO in S1008), the input/output control unit 307 ends the exception processing of the content mode (S1010).

### [Content Mode: Memory Content Display Processing]

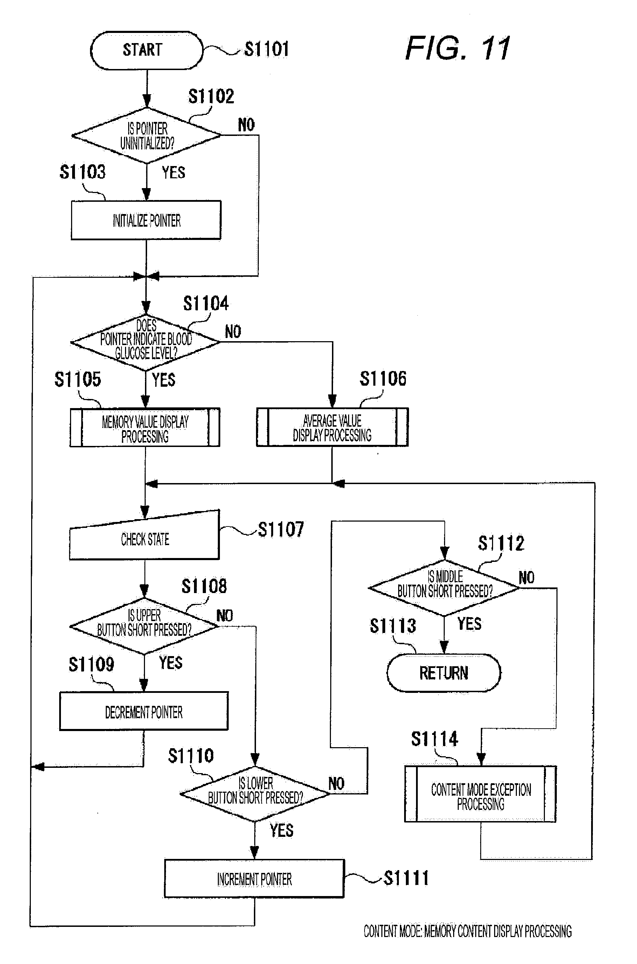

Fig. 11 is a flowchart illustrating an operation flow of the memory content display processing in the content mode of the blood glucose meter 101. Fig. 11 corresponds to step S809 in Fig. 8.

When the processing starts (S1101), the input/output control unit 307 determines whether or not the pointer 401 is uninitialized (S1102). If the pointer 401 is uninitialized (YES in S1102), the input/output control unit 307 initializes the pointer 401 (S1103).

In both cases in which the pointer 401 is initialized in step S1103 and the pointer 401 has been initialized in step S1102 (NO in S1102), the input/output control unit 307 sees content of the pointer 401 and determines whether or not the pointer 401 indicates a blood glucose level record in the blood glucose level table 306 (S1104). If the pointer 401 indicates a blood glucose level record (YES in S1104), the input/output control unit 307 performs memory value display processing which displays content of the blood glucose level record (S1105).

In step S1104, if the pointer 401 does not indicate a blood glucose level record (NO in S1104), the input/output control unit 307 performs average value display processing which displays an average value of the blood glucose levels in the last one month according to an average value display instruction indicated by the pointer 401 (S1106).

After performing the memory value display processing in step S1105 or the average value display processing in step S1106, the input/output control unit 307 checks the states of the operation buttons 104, the micro switch 210, and the RTC 204 (S1107).

First, the input/output control unit 307 determines whether or not the upper button 105 is short pressed (S1108). If the upper button 105 is short pressed (YES in S1108), the input/output control unit 307 decrements the value of the pointer 401 by one (S1109) and repeats the processing from step S1104 again.

In step S1108, if the upper button 105 is not short pressed (NO in S1108), the input/output control unit 307 determines whether or not the lower button 107 is short pressed (S1110). If the lower button 107 is short pressed (YES in S1110), the input/output control unit 307 increments the value of the pointer 401 by one (S1111) and repeats the processing from step S1104 again.

In step S1110, if the lower button 107 is not short pressed (NO in S1110), the input/output control unit 307 determines whether or not the middle button 106 is short pressed (S1112). If the middle button 106 is short pressed (YES in S1112), the input/output control unit 307 ends the memory content display processing of the content mode (S1113).

In step S1112, if the middle button 106 is not short pressed (NO in S1112), the input/output control unit 307 performs exception processing of the content mode (S1114) and checks again the states of the operation buttons 104, the micro switch 210, and the RTC 204 (S1107).

### [Content Mode: Memory Value Display Processing]

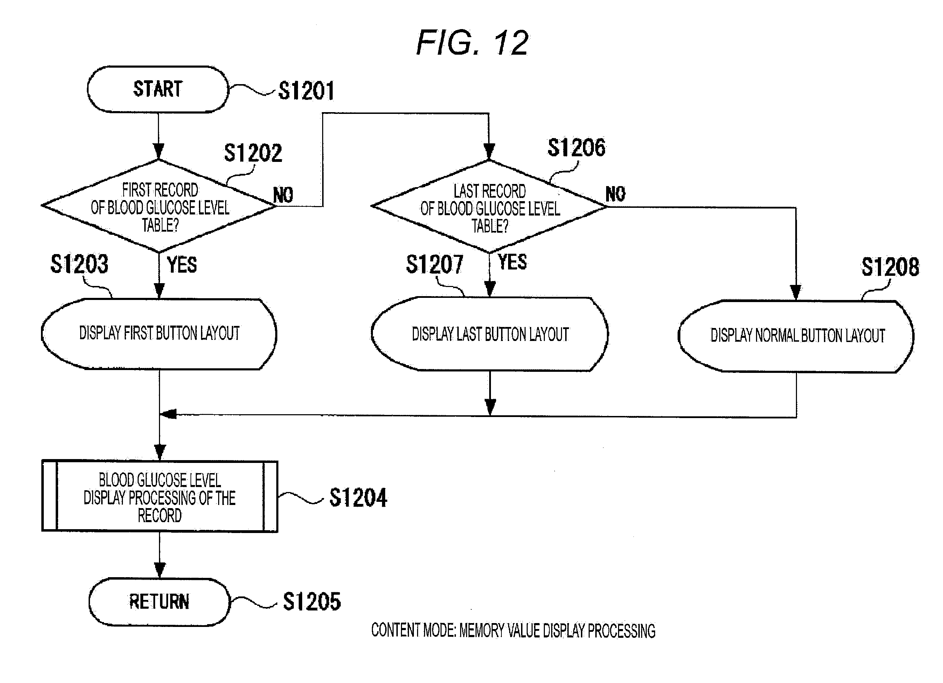

Fig. 12 is a flowchart illustrating an operation flow of the memory value display processing in the content mode of the blood glucose meter 101. Fig. 12 corresponds to step S1105 in Fig. 11.

When the processing starts (S1201), the input/output control unit 307 determines whether or not a record indicated by the pointer 401 is the first record of the blood glucose level table 306 (S1202). If the record indicated by the pointer 401 is the first record of the blood glucose level table 306 (YES in S1202), the input/output control unit 307 displays a button layout for the first record on the display unit 205 (S1203). Then, the input/output control unit 307 displays the blood glucose level of the record indicated by the pointer 401 on the display unit 205 (S1204) and ends the memory value display processing (S1205).

In step S1202, if the record indicated by the pointer 401 is not the first record of the blood glucose level table 306 (NO in S1202), the input/output control unit 307 determines whether or not the record indicated by the pointer 401 is the last record of the blood glucose level table 306 (S1206). If the record indicated by the pointer 401 is the last record of the blood glucose level table 306 (YES in S1206), the input/output control unit 307 displays a button layout for the last record on the display unit 205 (S1207). Then, the input/output control unit 307 displays the blood glucose level of the record indicated by the pointer 401 on the display unit 205 (S1204) and ends the memory value display processing (S1205).

In step S1206, if the record indicated by the pointer 401 is not the last record of the blood glucose level table 306 (NO in S1206), the input/output control unit 307 displays a button layout for a normal record on the display unit 205 (S1208). Then, the input/output control unit 307 displays the blood glucose level of the record indicated by the pointer 401 on the display unit 205 (S1204) and ends the memory value display processing (S1205).

### [Content Mode: Average Value Display Processing]

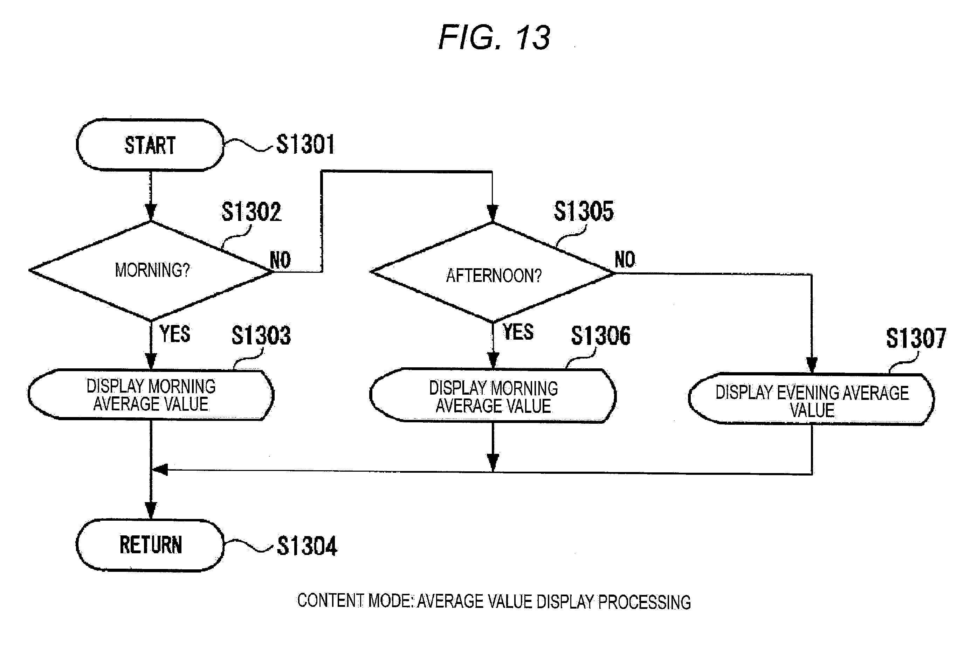

Fig. 13 is a flowchart illustrating an operation flow of the average value display processing in the content mode of the blood glucose meter 101. Fig. 13 corresponds to step S1106 in Fig. 11.

When the processing starts (S1301), the input/output control unit 307 determines whether or not a label indicated by the pointer 401 is an instruction to display an average value of morning blood glucose levels of the blood glucose levels recorded in the blood glucose level table 306 (S1302). If the label indicated by the pointer 401 is an instruction to display an average value of morning blood glucose levels (YES in S1302), the input/output control unit 307 displays the average value of morning blood glucose levels on the display unit 205 (S1303). Then, the input/output control unit 307 ends the average value display processing (S1304).

In step S1302, if the label indicated by the pointer 401 is not an instruction to display an average value of morning blood glucose levels (NO in S1302), the input/output control unit 307 determines whether or not the label indicated by the pointer 401 is an instruction to display an average value of afternoon blood glucose levels of the blood glucose levels recorded in the blood glucose level table 306 (S1305). If the label indicated by the pointer 401 is an instruction to display an average value of afternoon blood glucose levels (YES in S1305), the input/output control unit 307 displays the average value of afternoon blood glucose levels on the display unit 205 (S1306). Then, the input/output control unit 307 ends the average value display processing (S1304).

In step S1305, if the label indicated by the pointer 401 is not an instruction to display an average value of afternoon blood glucose levels (NO in S1305), the input/output control unit 307 displays an average value of evening blood glucose levels on the display unit 205 (S1307). Then, the input/output control unit 307 ends the average value display processing (S1304).

### [Content Mode: Display Screen in Memory Content Display Processing and Average Value Display Processing]

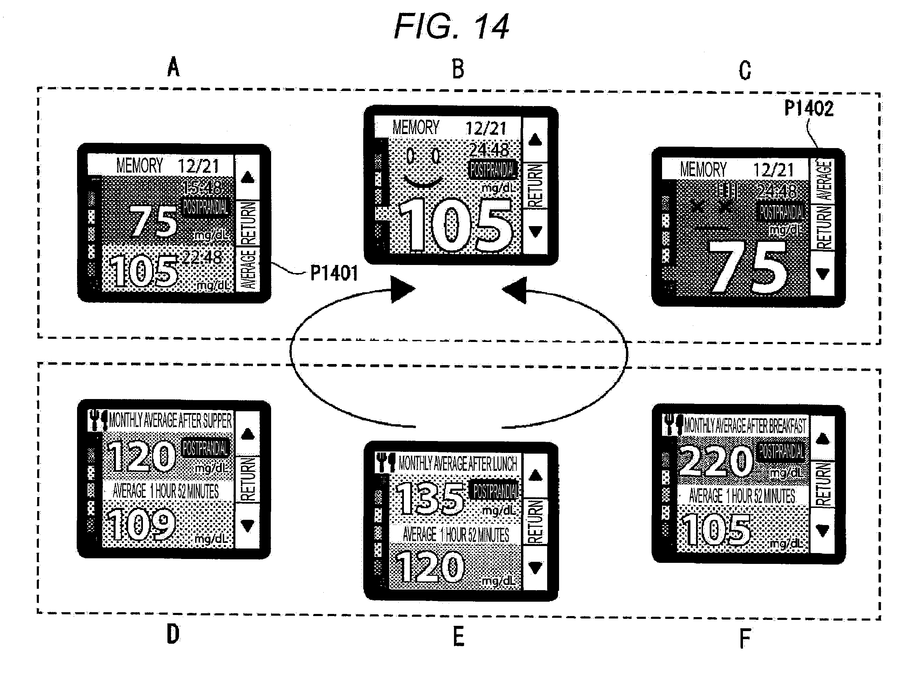

Figs. 14A, 14B, 14C, 14D, 14E, and 14F are schematic diagrams illustrating changes of the display screen in the memory content display processing in the content mode.

After the menu screen is displayed (step S903 or S909 in Fig. 8) in the content mode (Fig. 8), when the lower button 107 is short pressed (step S808 in Fig. 8) and the memory content display processing is selected (step S809 in Fig. 8), the input/output control unit 307 displays content of destination indicated by the pointer 401 on the display unit 205.

At the right side of the screen illustrated in Figs. 14A, 14B, 14C, 14D, 14E, and 14F, a guide related to the functions of the operation buttons 104 is displayed.

If the pointer 401 indicates the first record of the blood glucose level table 306 (S1202 in Fig. 12), the input/output control unit 307 displays the screen illustrated in Fig. 14A on the display unit 205 (steps S1203 and S1204 in Fig. 12). At the right side of the screen illustrated in Fig. 14A, a guide P1401 (a character string of "average" is represented) of the operation button 104 is displayed, which indicates that the screen moves to a screen of the average value display processing when the lower button 107 is pressed.

If the pointer 401 indicates the last record of the blood glucose level table 306 (S1206 in Fig. 12), the input/output control unit 307 displays the screen illustrated in Fig. 14C on the display unit 205 (steps S1207 and S1204 in Fig. 12). At the right side of the screen illustrated in Fig. 14C, a guide P1402 (a character string of "average" is represented) of the operation button 104 is displayed, which indicates that the screenmoves to the screen of the average value display processing when the upper button 105 is pressed.

If the pointer 401 indicates a record other than the first and the last records of the blood glucose level table 306, the input/output control unit 307 displays the screen illustrated in Fig. 14B on the display unit 205 (steps S1208 and S1204 in Fig. 12).

When the upper button 105 is pressed on the screen illustrated in Fig. 14C, the pointer 401 indicates a label representing the "average value of morning blood glucose levels" (step S1302 in Fig. 13). Therefore, the input/output control unit 307 displays the screen illustrated in Fig. 14F on the display unit 205 (step S1303 in Fig. 13).

When the upper button 105 is pressed on the screen illustrated in Fig. 14F, the pointer 401 indicates a label representing the "average value of afternoon blood glucose levels" (step S1305 in Fig. 13). Therefore, the input/output control unit 307 displays the screen illustrated in Fig. 14E on the display unit 205 (step S1306 in Fig. 13).

When the upper button 105 is pressed on the screen illustrated in Fig. 14E or the lower button 107 is pressed on the screen illustrated in Fig. 14A, the pointer 401 indicates a label representing the "average value of evening blood glucose levels". Therefore, the input/output control unit 307 displays the screen illustrated in Fig. 14F on the display unit 205 (step S1307 in Fig. 13).

As obvious from the above, the memory content display processing and the average value display processing are seamlessly performed by only operating the upper button 105 and the lower button 107.

### [Alarm Sounding Processing]

Fig. 15 is a flowchart illustrating an operation flow of the alarm sounding processing of the blood glucose meter 101. Fig. 15 corresponds to step S712 in Fig. 7 and is also performed from step S1009 in Fig. 10.

When the processing of step S712 in Fig. 7 is started (S1501) or when the processing of step S1009 in Fig. 10 is started (label "D"), the input/output control unit 307 sounds the buzzer 211 (alarm sounding) and sets the postprandial flag to logical "true" (S1502). Subsequently, the input/output control unit 307 displays a screen representing that an alarm is sounding on the display unit 205 (S1503). Then, the input/output control unit 307 checks the states of the operation buttons 104, the micro switch 210, and the RTC 204 (S1504).

First, the input/output control unit 307 determines whether or not the cap 108 is removed (S1505). If the cap 108 is removed (YES in S1505), the input/output control unit 307 stops the alarm (S1506) and moves to the measuring mode (from S1507 to the label "A" in Fig. 19; details will be described later with reference to Fig. 19).

In step S1505, if the cap 108 is not removed (NO in S1505), the input/output control unit 307 determines whether or not the middle button 106 is short pressed (S1508). If the middle button 106 is short pressed (YES in S1508), the input/output control unit 307 stops the alarm (S1509) and displays elapsed time from the alarm sounding time on the display unit 205 (S1510). Then, the input/output control unit 307 checks the states of the operation buttons 104, the micro switch 210, and the RTC 204 (S1511).

Subsequently, the input/output control unit 307 determines whether or not the middle button 106 is short pressed (S1512). If the middle button 106 is short pressed (YES in S1512), the input/output control unit 307 moves to the content mode (from S1513 to the label "B" in Fig. 8).

In step S1512, if the middle button 106 is not short pressed (NO in S1512), the input/output control unit 307 performs exception processing of the content mode (S1514) and checks again the states of the operation buttons 104, the micro switch 210, and the RTC 204 (S1511).

In step S1508, if the middle button 106 is not short pressed (NO in S1508), the input/output control unit 307 performs exception processing of the content mode (S1515) and checks again the states of the operation buttons 104, the micro switch 210, and the RTC 204 (S1504).

### [Postprandial Flag Operation Processing and Operation Timer 308 Restart Processing]

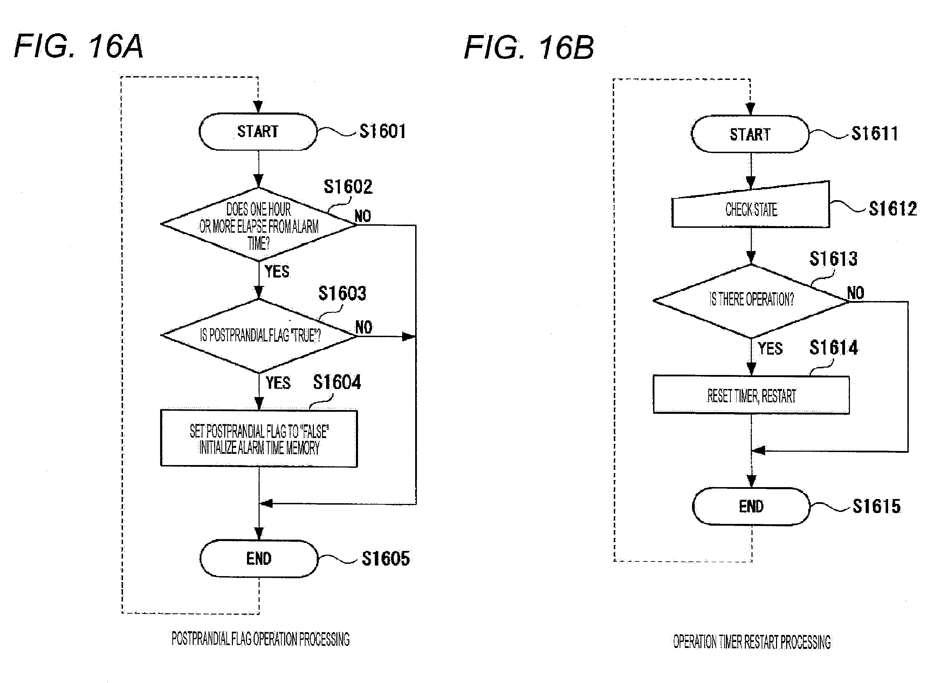

Figs. 16A and 16B are flowcharts illustrating processing that runs at all times when the blood glucose meter 101 is in any of the content mode, the measuring mode, and the setting mode (that is, in a mode other than the power saving mode) regardless of the operation mode.

Fig. 16A is a flowchart illustrating postprandial flag operation processing.

When the processing starts (S1601), the input/output control unit 307 determines whether or not one hour or more has elapsed from the alarm time to the current time by seeing the alarm time stored in the alarm time memory 309 (S1602).

If one hour or more has elapsed from the alarm time to the current time (YES in S1602), the input/output control unit 307 determines whether or not the postprandial flag is set to logical "true" (S1603).

If the postprandial flag is set to logical "true" (YES in S1603), the input/output control unit 307 sets the postprandial flag to logical "false", initializes the alarm time memory 309 (S1604), and ends a series of processing steps (S1605).

When one hour or more has not elapsed from the alarm time to the current time or the alarm time memory 309 has already been initialized in step S1602 (NO in S1602) and when the postprandial flag is set to logical "false" in step S1603 (NO in S1603), the input/output control unit 307 does nothing and ends the processing (S1605).

The postprandial flag operation processing is repeatedly performed (from S1605 to S1601) at all times in a mode other than the power saving mode.

Fig. 16B is a flowchart illustrating operation timer 308 restart processing.

When the processing starts (S1611), the input/output control unit 307 checks the states of the operation buttons 104, the micro switch 210, and the blood glucose level measuring unit 301 (S1612). If the operation button 104 is operated, or the state of the micro switch 210 changes, or the blood glucose level measuring unit 301 performs blood glucose level measuring processing (YES in S1613), the input/output control unit 307 resets and restarts the operation timer 308 (S1614) and ends a series of processing steps (S1615).

The operation timer 308 restart processing is also repeatedly performed (from S1615 to S1611) at all times in a mode other than the power saving mode.

### [Postprandial Alarm Display Screen]

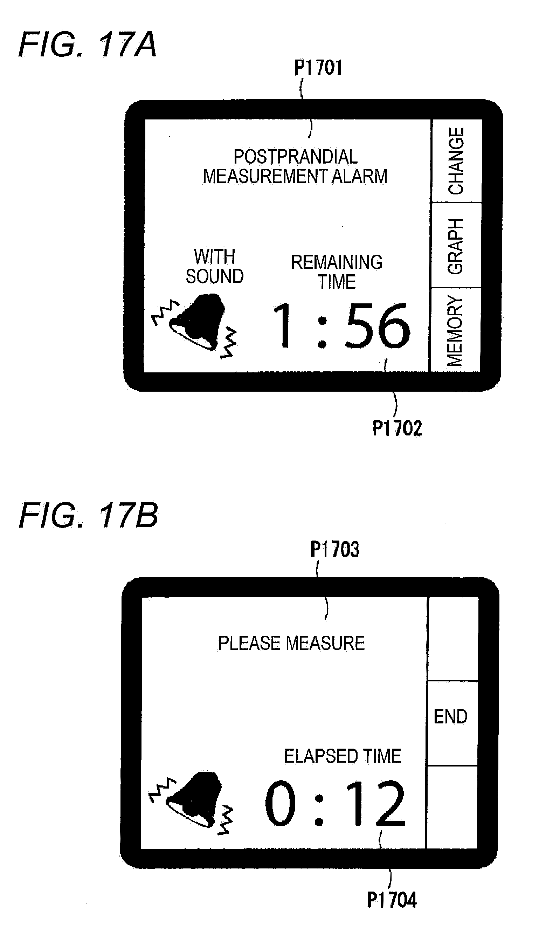

Figs. 17A and 17B are diagrams illustrating a display screen of the postprandial alarm.

Fig. 17A is a screen displayed on the liquid crystal display 103 (the display unit 205) when the alarm sounding time is within two hours from the current time. Fig. 17A is a screen displayed in step S903 in Fig. 9. In the screen, a message P1701 indicating that the postprandial alarm is set and a remaining time P1702 before the postprandial alarm sounding time are displayed.

Fig. 17B is a screen displayed on the liquid crystal display 103 (the display unit 205) when the current time is within one hour from the alarm sounding time. Fig. 17B is a screen displayed in step S906 in Fig. 9. In the screen, a message P1703 prompting to measure the blood glucose level and an elapsed time P1704 from the postprandial alarm sounding time are displayed.

An effect of improving self-management awareness of a patient can be expected by displaying the elapsed time from the postprandial alarm sounding time in this manner.

When the screen illustrated in Fig. 17B is displayed (YES in step S905 in Fig. 9), the screen is also a screen indicating that the postprandial flag is set to logical "true" (NO in step S1602 in Fig. 16), so that the patient can smoothly perform a postprandial blood glucose level measuring operation by clearly recognizing the postprandial blood glucose level measuring operation.

### [Setting Mode]

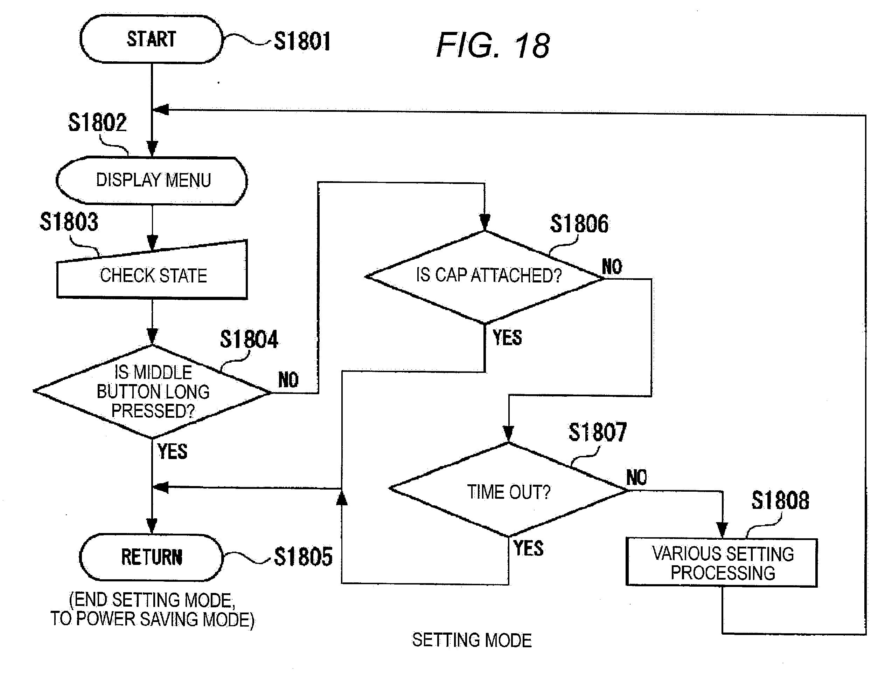

Fig. 18 is a flowchart illustrating an operation flow of the setting mode of the blood glucose meter 101. Fig. 18 corresponds to step S710 in Fig. 7.

When the processing starts (S1801), the input/output control unit 307 first displays a menu screen for the setting mode on the display unit 205 (S1802).

Subsequently, the input/output control unit 307 checks the states of the operation buttons 104, the micro switch 210, and the operation timer 308 (S1803). As a result, if the middle button 106 is long pressed (YES in S1804), the input/output control unit 307 ends the setting mode (S1805). Thereby, the blood glucose meter 101 moves to the power saving mode (step S702 in Fig. 7).

In step S1804, if the middle button 106 is not long pressed (NO in S1804), the input/output control unit 307 sees the state of the micro switch 210 and determines whether or not the cap 108 is attached (S1806). If the cap 108 is attached (YES in S1806), the input/output control unit 307 ends the processing of the setting mode (S1805). Thereby, the blood glucose meter 101 moves to the power saving mode (step S702 in Fig. 7).

In step S1806, if the cap 108 is not attached (NO in S1806), the input/output control unit 307 sees the state of the operation timer 308 and determines whether or not the operation timer 308 reaches timeout (S1807). If the operation timer 308 reaches timeout (YES in S1807), the input/output control unit 307 ends the processing of the setting mode (S1805). Thereby, the blood glucose meter 101 moves to the power saving mode (step S702 in Fig. 7).

In step S1807, if the operation timer 308 does not reach timeout (NO in S1807), the input/output control unit 307 performs various setting processing (S1808) according to operation information outputted by the operation unit 206 and repeats the processing from step S1802 again.

### [Measuring Mode]

Figs. 19 and 20 are flowcharts illustrating an operation flow of the measuring mode of the blood glucose meter 101. The flowcharts of Figs. 19 and 20 correspond to step S708 in Fig. 7 and are also performed from step S1007 in Fig. 10.

When the processing of step S708 in Fig. 7 is started (S1901) or when the processing of step S708 in Fig. 7 is started or when the processing of step S1007 in Fig. 10 is started (label "A"), the input/output control unit 307 first performs button area display processing (S1902) which displays a button area adj acent to the operation buttons 104 in the screen displayed on the display unit 205. Subsequently, the input/output control unit 307 displays a chip attaching guide screen that guides a patient to attach a chip to the blood glucose meter 101, which is a first stage of the blood glucose level measuring operation (S1903).

Subsequently, the input/output control unit 307 checks the states of the operation buttons 104, the micro switch 210, the operation timer 308, and the photodiode 215 (S1904). As a result, if the input/output control unit 307 cannot detect that the chip is normally attached to the blood glucose meter 101 (NO in S1905), the input/output control unit 307 performs exception processing of the measuring mode (S1906) and repeats the processing from step S1902 again.

In step S1905, if the input/output control unit 307 can detect that the chip is normally attached to the blood glucose meter 101 (YES in S1905), the input/output control unit 307 moves to processing for spotting blood 304 to the chip, which is the next stage of the blood glucose level measuring operation.

First, in the same manner as in step S1902, the input/output control unit 307 performs the button area display processing (S1907). Subsequently, the input/output control unit 307 displays a blood spotting guide screen that guides the patient to spot the blood 304 to the chip, which is the next stage of the blood glucose level measuring operation (S1908).

Subsequently, the input/output control unit 307 checks the states of the operation buttons 104, the micro switch 210, the operation timer 308, and the photodiode 215 (S1909). As a result, if the input/output control unit 307 cannot detect that the blood 304 is normally spotted to the chip (NO in S1910), the input/output control unit 307 performs exception processing of the measuring mode (S1911) and repeats the processing from step S1907 again.

In step S1910, if the input/output control unit 307 can detect that the blood 304 is normally spotted to the chip (YES in S1910), the input/output control unit 307 moves to processing for measuring the blood glucose level, which is the next stage of the blood glucose level measuring operation.

First, the input/output control unit 307 starts the measurement timer 303 for measuring the blood glucose level

(S1912). The measurement timer 303 times, for example, nine seconds. Thereafter, the processing continues to Fig. 20 (from S1913 to the label "C" in Fig. 20).

The continuation of the flowchart will be described with reference to Fig. 20.

Subsequently, in the same manner as in steps S1902 and S1907, the input/output control unit 307 performs the button area display processing (S2014). Subsequently, the input/output control unit 307 displays a being-measured display screen that guides the patient to wait until the blood glucose level measuring operation is completed, that is, until the measurement timer 303 completes to time a measurement time (nine seconds in the present embodiment), which is the next stage of the blood glucose level measuring operation (S2015).

Subsequently, the input/output control unit 307 checks the states of the operation buttons 104, the micro switch 210, the measurement timer 303, and the operation timer 308 (S2016). As a result, if the measurement timer 303 does not complete to time the measurement time (NO in S2017), the input/output control unit 307 checks whether or not the upper button 105 is short pressed (S2018).

If the upper button 105 is short pressed (YES in S2018), the input/output control unit 307 sets the state of the postprandial flag to logical "true" (S2019) and repeats the processing from step S2014 again. In other words, the input/output control unit 307 sets the state of the postprandial flag to a logical value indicating postprandial.

In step S2018, if the upper button 105 is not short pressed (NO in S2018), the input/output control unit 307 checks whether or not the upper button 105 is long pressed (S2020).

If the upper button 105 is long pressed (YES in S2020), the input/output control unit 307 sets the state of the postprandial flag to logical "false" (S2021) and repeats the processing from step S2014 again. In other words, the input/output control unit 307 sets the state of the postprandial flag to a logical value indicating preprandial.

In step S2020, if the upper button 105 is not long pressed (NO in S2020), the input/output control unit 307 repeats the processing from step S2014 again.

In step S2017, if the measurement timer 303 completes to time the measurement time (YES in S2017), the input/output control unit 307 causes the blood glucose level calculation unit 302 to calculate the blood glucose level (S2022).

Subsequently, in the same manner as in steps S1902, S1907, and S2014, the input/output control unit 307 performs the button area display processing (S2023). Subsequently, the input/output control unit 307 performs blood glucose level display processing for displaying the blood glucose level on the display unit 205 (S2024). Then, the input/output control unit 307 checks the states of the operation buttons 104, the micro switch 210, and the operation timer 308 (S2025).

Subsequently, the input/output control unit 307 performs blood glucose level recording processing which records the blood glucose level calculated in step S2022 in the blood glucose level table 306 along with the postprandial flag (S2026). Then, the input/output control unit 307 performs exception processing of the measuring mode (S2027).

After performing the blood glucose level recording processing in step S2026 and the exception processing of the measuring mode in step S2027 in this manner, the input/output control unit 307 determines whether or not operation information obtained from the operation buttons 104 is an operation to move to the content mode (S2028). If the operation information is an operation to move to the content mode (YES in S2028), the input/output control unit 307 moves to the content mode (from S2029 to the label "B" in Fig. 8).

In step S2028, if the operation information obtained from the operation buttons 104 is not an operation to move to the content mode (NO in S2028), the input/output control unit 307 repeats the processing from step S2023 again.

### [Measuring Mode: Exception Processing]

Fig. 21 is a flowchart illustrating an operation flow of the exception processing of the measuring mode of the blood glucose meter 101. Fig. 21 corresponds to steps S1906 and S1911 in Fig. 19 and step S2025 in Fig. 20.

When the processing starts (S2101), the input/output control unit 307 determines whether or not the middle button 106 is long pressed (S2102). If the middle button 106 is long pressed (YES in S2102), the input/output control unit 307 ends the processing of the measuring mode (S2103). Thereby, the blood glucose meter 101 moves to the power saving mode (step S702 in Fig. 7).

In step S2102, if the middle button 106 is not long pressed (NO in S2102), the input/output control unit 307 sees the state of the micro switch 210 and determines whether or not the cap 108 is attached (S2104). If the cap 108 is attached (YES in S2104), the input/output control unit 307 ends the processing of the measuring mode (S2103). Thereby, the blood glucose meter 101 moves to the power saving mode (step S702 in Fig. 7).

In step S2104, if the cap 108 is not attached (NO in S2104), the input/output control unit 307 sees the operation timer 308 and determines whether or not the operation timer 308 reaches timeout (S2105). If the operation timer 308 reaches timeout (YES in S2105), the input/output control unit 307 ends the processing of the measuring mode (S2103). Thereby, the blood glucose meter 101 moves to the power saving mode (step S702 in Fig. 7).

In step S2105, if the operation timer 308 does not reach timeout (NO in S2105), the input/output control unit 307 determines whether or not operation information obtained from the operation buttons 104 is an operation to move to the content mode (S2106). If the operation information is an operation to move to the content mode (YES in S2106), the input/output control unit 307 moves to the content mode (from S2107 to the label "B" in Fig. 8).

In step S2106, if the operation information obtained from the operation buttons 104 is not an operation to move to the content mode (NO in S2106), the input/output control unit 307 determines whether or not the upper button 105 is short pressed (S2108).

If the upper button 105 is short pressed (YES in S2108), the input/output control unit 307 sets the state of the postprandial flag to logical "true" (S2109). In other words, the input/output control unit 307 sets the state of the postprandial flag to a logical value indicating postprandial. Then, the input/output control unit 307 ends the exception processing of the content mode (S2110).

In step S2108, if the upper button 105 is not short pressed (NO in S2108), the input/output control unit 307 determines whether or not the upper button 105 is long pressed (S2111).

If the upper button 105 is long pressed (YES in S2111), the input/output control unit 307 sets the state of the postprandial flag to logical "false" (S2112). In other words, the input/output control unit 307 sets the state of the postprandial flag to a logical value indicating preprandial. Then, the input/output control unit 307 ends the exception processing of the content mode (S2110).

In step S2111, if the upper button 105 is not long pressed (NO in S2111), the input/output control unit 307 ends the exception processing of the content mode (S2110).

### [Measuring Mode: Blood Glucose Level Recording Processing]

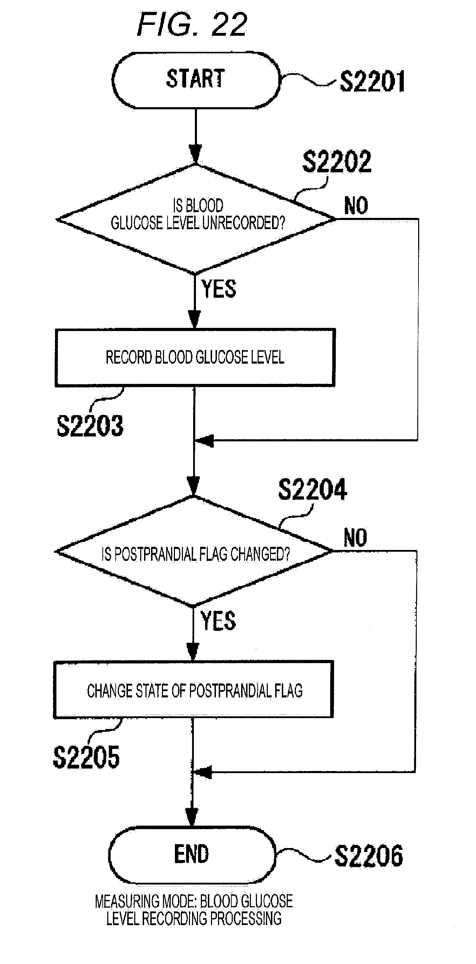

Fig. 22 is a flowchart illustrating an operation flow of the blood glucose level recording processing of the measuring mode of the blood glucose meter 101. Fig. 22 corresponds to step S2024 in Fig. 19.

When the processing starts (S2201), the input/output control unit 307 determines whether or not the blood glucose level calculated in step S2020 in Fig. 20 has not been recorded in the blood glucose level table 306 (S2202). If the calculated blood glucose level has not been recorded (YES in S2202), the input/output control unit 307 records the calculated blood glucose level in the blood glucose level table 306 along with the date and time when the blood glucose level is measured and the postprandial flag (S2203).

In step S2202, if the blood glucose level has been recorded (NO in S2202), the input/output control unit 307 does not record the calculated blood glucose level in the blood glucose level table 306.

Subsequently, the input/output control unit 307 determines whether or not the logical value of the current postprandial flag is not coincident with the postprandial flag recorded in the blood glucose level table 306, that is, whether or not the postprandial flag is changed (S2204). If it is determined that the postprandial flag is changed (YES in S2204), the input/output control unit 307 writes the logical value of the current postprandial flag to the postprandial field of the record where the current blood glucose level is recorded in the blood glucose level table 306 (S2205).

In step S2204, if it is not determined that the postprandial flag is changed (NO in S2204), the input/output control unit 307 does not record the postprandial flag in the blood glucose level table 306.

In both cases in which the input/output control unit 307 records the postprandial flag (S2205) and does not record the postprandial flag (NO in S2204) in the blood glucose level table 306, the input/output control unit 307 ends a series of processing steps (S2206).

### [Measuring Mode: Button Area Display Processing]

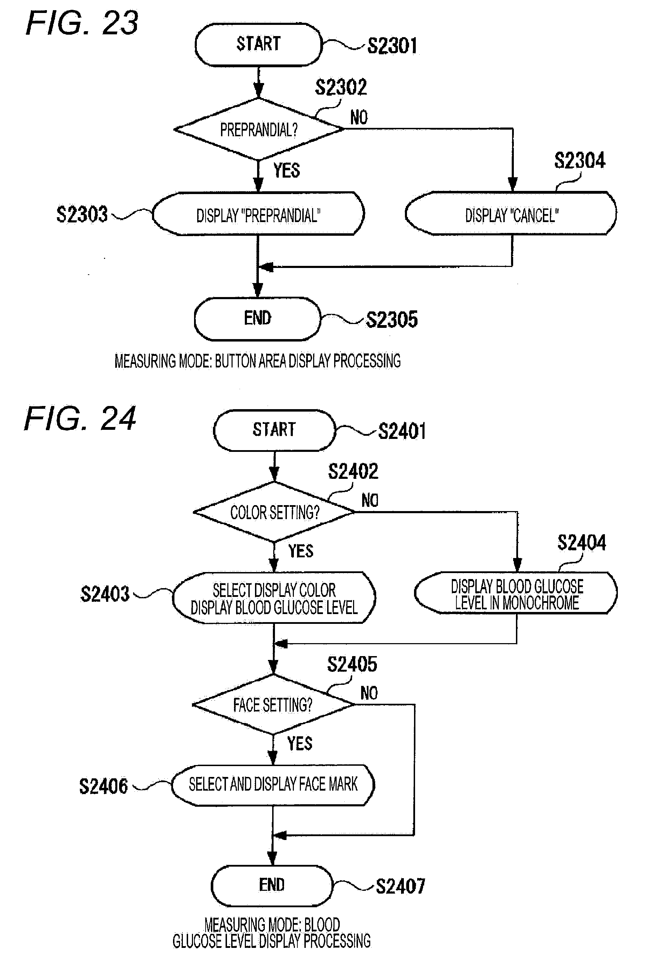

Fig. 23 is a flowchart illustrating an operation flow of the button area display processing of the measuring mode of the blood glucose meter 101. Fig. 23 corresponds to steps S1902 and S1907 in Fig. 19 and steps S2014 and S2023 in Fig. 20.

When the processing starts (S2301), the input/output control unit 307 sees the state of the postprandial flag and determines whether or not the logical value of the postprandial flag indicates "preprandial" (S2302).

If the logical value of the postprandial flag indicates "preprandial" (YES in S2302), the input/output control unit 307 displays a character string of "preprandial" in a region adj acent to the upper button 105 in the button area at the right end of the display unit 205 (S2303).

If the logical value of the postprandial flag indicates "postprandial" (NO in S2302), the input/output control unit 307 displays a character string of "cancel" in a region adjacent to the upper button 105 in the button area at the right end of the display unit 205 (S2304).

After any one of steps S2303 and S2304, the input/output control unit 307 ends a series of processing steps (S2305).

### [Content Mode and Measuring Mode: Blood Glucose Level Display Processing]

Fig. 24 is a flowchart illustrating an operation flow of the blood glucose level display processing of the content mode and the measuring mode of the blood glucose meter 101. Fig. 24 corresponds to step S1204 in Fig. 12 and step S2022 in Fig. 20.

When the processing starts (S2401), the input/output control unit 307 sees the various setting information and determines whether or not the setting information of the screen displayed on the display unit 205 is a color setting (S2402).

If the setting information of the screen displayed on the display unit 205 is a color setting (YES in S2402), the input/output control unit 307 performs five-level evaluation of the blood glucose level to be displayed by comparing the blood glucose level with a predetermined threshold value, selects a display color based on a result of the five-level evaluation, and displays the blood glucose level on the display unit 205 (S2403).

If the setting information of the screen displayed on the display unit 205 is a monochrome setting (NO in S2402), the input/output control unit 307 displays the blood glucose level to be displayed on the display unit 205 by using a monochrome screen (S2404).

After step S2403 or step S2404, the input/output control unit 307 sees the various setting information and determines whether or not the setting information of the screen displayed on the display unit 205 is a setting to display a face mark (S2405).

If the setting information of the screen displayed on the display unit 205 is a setting to display a face mark (YES in S2405), the input/output control unit 307 performs five-level evaluation of the blood glucose level to be displayed on the display unit 205 by comparing the blood glucose level with a predetermined threshold value, selects a face mark based on a result of the five-level evaluation, and displays the blood glucose level on the display unit 205 (S2406).

If the setting information of the screen displayed on the display unit 205 is not a setting to display a face mark (NO in S2405), the input/output control unit 307 does not display a face mark on the display unit 205.

After NO in any one of steps S2406 and S2405, the input/output control unit 307 ends a series of processing steps (S2407).

### [Measuring Mode: Display Screen in Blood Glucose Level Display Processing]

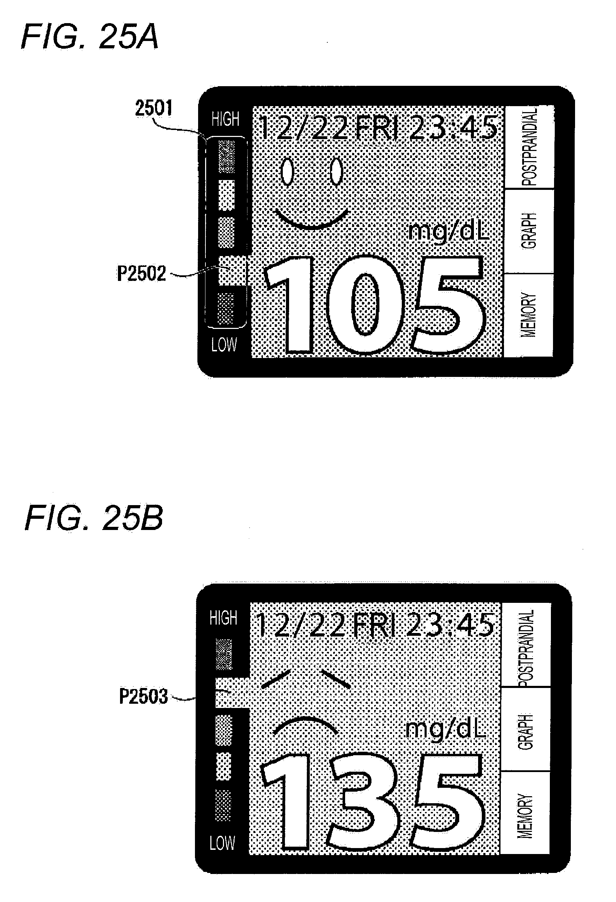

Figs. 25A and 25B are display screens displayed on the display unit 205 in the blood glucose level display processing of the measuring mode of the blood glucose meter 101. More specifically, Figs. 25A and 25B are display screens displayed in step S2403 and step S2406 in Fig. 24.

When the various setting information is a color setting, the blood glucose level is color-coded by the five-level evaluation and displayed on the display unit 205. When sorted in ascending order, the lowest blood glucose level is blue, the optimal blood glucose level is yellow-green, the next blood glucose level is orange, the next blood glucose level is pink, and the highest blood glucose level is red.

Fig. 25A is a screen displayed on the display unit 205 when the blood glucose level is lower than or equal to the second lowest blood glucose level of the five-level evaluation. The display screen is displayed in yellow-green of the optimal blood glucose level and is in a display state in which the yellow-green is integrated with a second lowest yellow-green portion of a color-coded scale 2501 displayed at the left end of the screen. The color-coded scale 2501 and the background of the screen are integrated together, so that a tab P2502 is formed on the display screen.

Fig. 25B is a screen displayed on the display unit 205 when the blood glucose level is higher than or equal to the second highest blood glucose level of the five-level evaluation. The display screen is displayed in pink indicating that the blood glucose level is the second highest blood glucose level and is in a display state in which the pink is integrated with a second highest pink portion of the color-coded scale 2501 displayed at the left end of the screen and a tab P2503 is formed.

In this way, a tab is provided, which is formed by integrating the background of the screen with the color-coded scale 2501, so that it is possible to instantly and clearly recognize how high or how low the current blood glucose level is in the five-level evaluation.

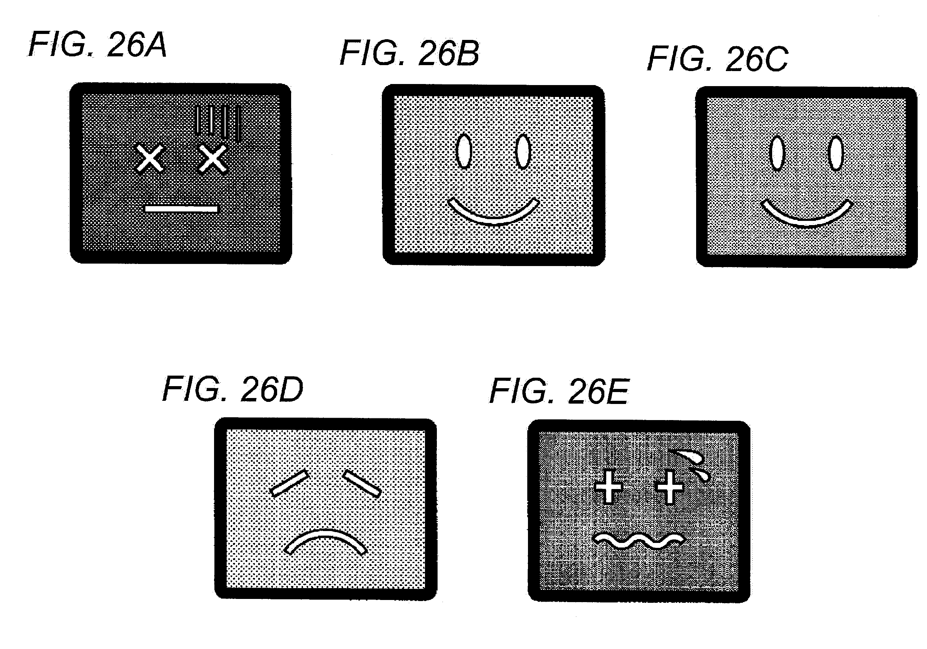

Figs. 26A, 26B, 26C, 26D, and 26E are diagrams illustrating a variation of a face mark displayed on the display unit 205 in the blood glucose level display processing of the content mode and the measuring mode of the blood glucose meter 101. Figs. 26A, 26B, 26C, 26D, and 26E are face marks displayed on the display unit 205 according to the blood glucose level when the setting information of the screen displayed on the display unit 205 is a setting to display a face mark (YES in S2405) in step S2405 in Fig. 24.

The input/output control unit 307 performs five-level evaluation of the blood glucose level to be displayed by comparing the blood glucose level with a predetermined threshold value, selects a face mark based on a result of the five-level evaluation, and displays the blood glucose level on the display unit 205 (S2406).

In the five-level evaluation, the face marks illustrated in Figs. 26A, 26B, 26C, 26D, and 26E are displayed by using threshold values based on the Japan Diabetes Society "Guideline for the Treatment for Diabetes"
(http://www.lifescience.jp/ebm/cms/ms/no.19/topics.pdf).

Fig. 26A is a face mark indicating a low blood glucose level. This face mark is displayed when a fasting blood glucose level and a two-hour postprandial blood glucose level is lower than 80 mg/dl (deciliter).

Fig. 26B is a face mark indicating an appropriate blood glucose level. This face mark is displayed when the fasting blood glucose level is in a range from 80 to 110 mg/dl and the two-hour postprandial blood glucose level is in a range from 80 to 140 mg/dl.

In other words, the blood glucose meter 101 of the present embodiment employs different values for preprandial and postprandial values, respectively, as the threshold values used for the five-level evaluation.

Fig. 26C is a face mark indicating that the blood glucose level is a little high. This face mark is displayed when the fasting blood glucose level is higher than or equal to 110 mg/dl and lower than 130 mg/dl and the two-hour postprandial blood glucose level is higher than or equal to 140 mg/dl and lower than 180 mg/dl.

Fig. 26D is a face mark indicating that the blood glucose level is high. This face mark is displayed when the fasting blood glucose level is higher than or equal to 130 mg/dl and lower than 160 mg/dl and the two-hour postprandial blood glucose level is higher than or equal to 180 mg/dl and lower than 220 mg/dl.

Fig. 26E is a face mark indicating that the blood glucose level is too high and dangerous. This face mark is displayed when the fasting blood glucose level is higher than or equal to 160 mg/dl and the two-hour postprandial blood glucose level is higher than or equal to 220 mg/dl.

In this manner, the face marks having different facial expressions illustrated in Figs. 26A, 26B, 26C, 26D, and 26E correspond to blood glucose levels respectively. The blood glucose meter 101 of the present embodiment converts the blood glucose level into one of the face marks having different facial expressions and displays the face mark on the display unit 205, so that it is possible to instantly and clearly recognize how high or how low the current blood glucose level is in the five-level evaluation.

### [Content Mode and Measuring Mode: Display Screen]

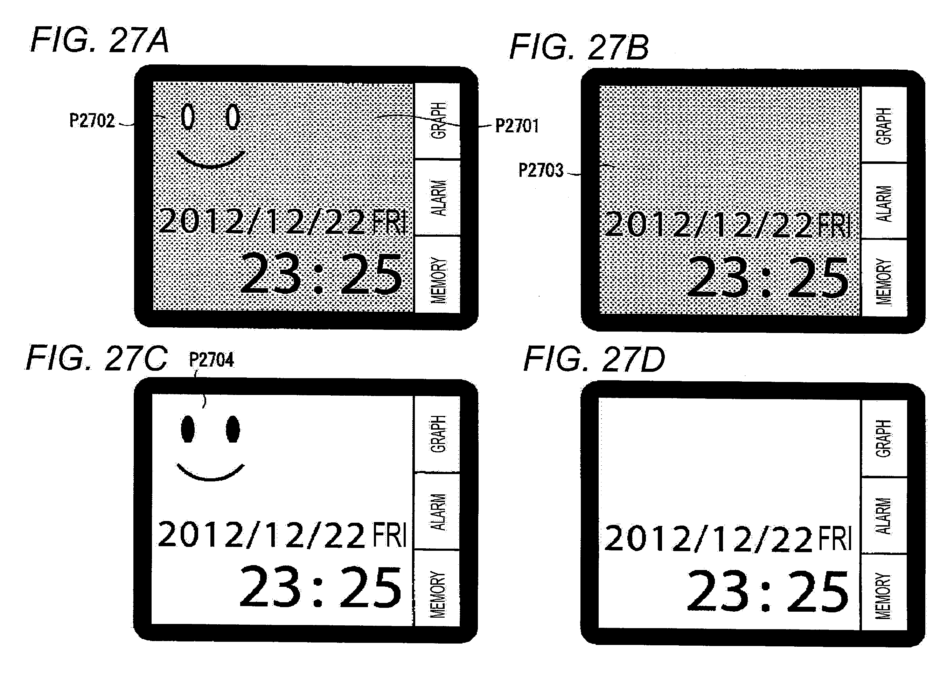

Figs. 27A, 27B, 27C, and 27D are diagrams illustrating a variation of the display screen displayed on the display unit 205 in the blood glucose level display processing of the content mode and the measuring mode of the blood glucose meter 101. More specifically, Figs. 27A, 27B, 27C, and 27D are display screens displayed in step S1204 in Fig. 12.

Fig. 27A is a display screen displayed when the various setting information is a color setting (YES in S2402) and a face mark setting (YES in S2405). The screen is displayed with a background color P2701 of the five-level evaluation and a face mark P2702 based on the five-level evaluation is displayed on the screen.

Fig. 27B is a display screen displayed when the various setting information is a color setting (YES in S2402) and no face mark setting (NO in S2405). The screen is displayed with a background color P2703 of the five-level evaluation. However, a face mark based on the five-level evaluation is not displayed on the screen.

Fig. 27C is a display screen displayed when the various setting information is a monochrome setting (YES in S2402) and a face mark setting (YES in S2405). The screen is displayed in monochrome and a face mark P2704 based on the five-level evaluation is displayed on the screen.

Fig. 27D is a display screen displayed when the various setting information is a monochrome setting (YES in S2402) and no face mark setting (NO in S2405). The screen is displayed in monochrome and a face mark based on the five-level evaluation is not displayed on the screen.

In this manner, the form of the screen displayed on the display unit 205 changes according to the setting content of the various setting information.

While Figs. 27A, 27B, 27C, and 27D illustrate a variation of the display screen in the contentmode, there is also a variation of the display screen in the measuring mode.

The color display which is designed for a patient to easily see the screen is difficult to be seen by the patient if the patient develops a visual impairment due to complications such as diabetic retinopathy depending on a combination of colors of the display screen displayed on the display unit 205. Therefore, a setting function to switch the display to a high contrast display such as a monochrome display and a setting function to switch the presence or absence of the mark display are given to the blood glucose meter 101, so that it is possible to select an optimal blood glucose level display on the display unit 205 according to the progression of the visual impairment.

### [Postprandial Button of Measuring Mode and Postprandial Alarm Button of Content Mode]

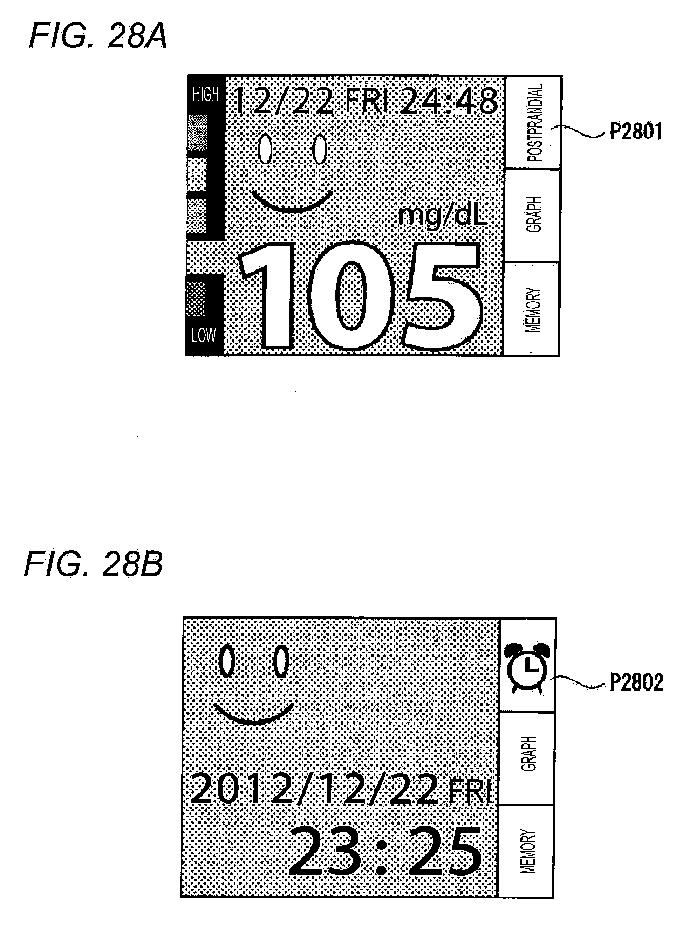

Figs. 28A and 28B are display screens in the measuring mode and a display screen in the content mode.

In Figs. 28A and 28B, a button display area that explains the functions of the operation buttons 104 arranged adjacent to the button display area is provided at the right end of the screen.

In the button display area in Fig. 28A, a character string of "postprandial" is displayed in an area P2801 of the upper button 105. A function of postprandial button is assigned to the upper button 105 in the measuring mode and the postprandial flag is changed by short pressing the upper button 105.

In the button display area in Fig. 28B, an illustration of a clock is displayed in an area P2802 of the upper button 105. A function to set a postprandial alarm is assigned to the upper button 105 in the content mode and a menu screen to set a postprandial alarm is displayed by short pressing the upper button 105.

### [Function Assignment of Operation Buttons 104]

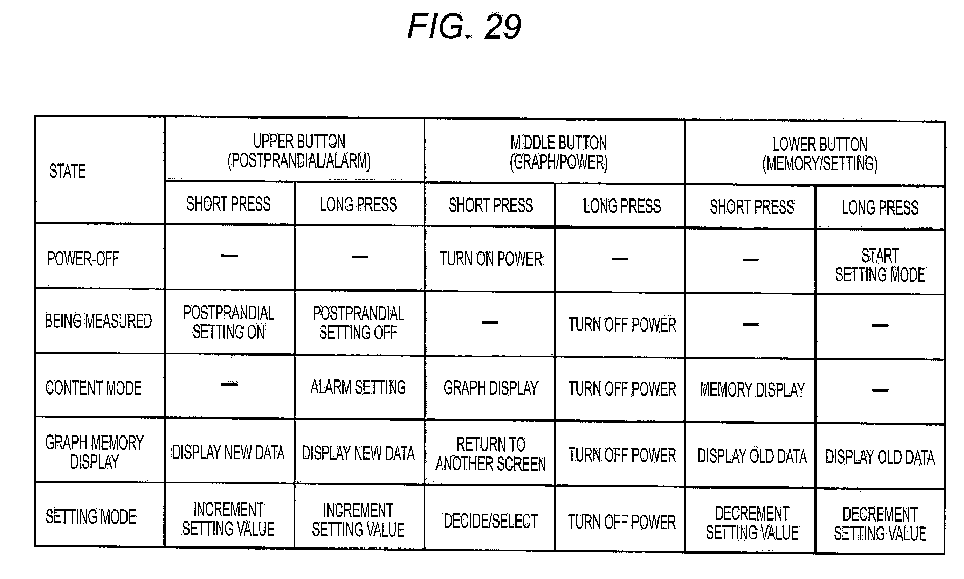

Fig. 29 is a table illustrating functions assigned to the operation buttons 104.

With reference to Figs. 7 to 24, main functions assigned to the operation buttons 104 have been described by using the flowcharts. The table in Fig. 29 is a table illustrating a relationship between the operation buttons 104 and a function assigned for each operation mode.

In a state of power off, that is, the power saving mode, the input/output control unit 307 detects only the short press of the middle button 106 and the long press of the lower button 107.

When the middle button 106 is short pressed, the input/output control unit 307 moves to the content mode. In other words, the input/output control unit 307 is in the power-on state.

When the lower button 107 is long pressed, the input/output control unit 307 moves to the setting mode.

Although not illustrated in the table, the removal of the cap 108 is detected through the micro switch 210. When the cap 108 is removed, the input/output control unit 307 moves to the measuring mode.

In a state of the measuring mode, the input/output control unit 307 detects only the short press and the long press of the upper button 105 and the long press of the middle button 106.

When the upper button 105 is short pressed, the input/output control unit 307 sets the postprandial flag to logical "true". In other words, the input/output control unit 307 sets a postprandial setting to ON.

When the upper button 105 is long pressed, the input/output control unit 307 sets the postprandial flag to logical "false". In other words, the input/output control unit 307 sets the postprandial setting to OFF.

When the middle button 106 is long pressed, the input/output control unit 307 moves to the power saving mode. In other words, the power-off state is achieved.

In a state of the content mode, the input/output control unit 307 detects only the long press of the upper button 105, the short press and the long press of the middle button 106, and the short press of the lower button 107.

When the upper button 105 is long pressed, the input/output control unit 307 starts an alarm setting function.

When the middle button 106 is short pressed, the input/output control unit 307 starts a graph display function.

When the middle button 106 is long pressed, the input/output control unit 307 moves to the power saving mode. In other words, the power-off state is achieved.

When the lower button 107 is short pressed, the input/output control unit 307 starts a memory display function.

In a state of the graph display function and the memory display function, the input/output control unit 307 detects the short press and the long press of the upper button 105, the short press and the long press of the middle button 106, and the short press and the long press of the lower button 107, that is, the short press and the long press of all the buttons.

When the upper button 105 is short pressed, the input/output control unit 307 moves the pointer 401, which indicates data that is currently displayed, to new data and displays the new data on the display unit 205.

When the upper button 105 is long pressed, the input/output control unit 307 moves the pointer 401, which indicates data that is currently displayed, to new data and displays the new data on the display unit 205. In this operation, if possible, the input/output control unit 307 continuously displays data on the display unit 205.

When the middle button 106 is short pressed, the input/output control unit 307 returns to a previous screen from which the function that is currently performed is called.

When the middle button 106 is long pressed, the input/output control unit 307 moves to the power saving mode. In other words, the power-off state is achieved.

When the lower button 107 is short pressed, the input/output control unit 307 moves the pointer 401, which indicates data that is currently displayed, to old data and displays the old data on the display unit 205.

When the lower button 107 is long pressed, the input/output control unit 307 moves the pointer 401, which indicates data that is currently displayed, to old data and displays the old data on the display unit 205. In this operation, if possible, the input/output control unit 307 continuously displays data on the display unit 205.

In a state of the setting mode, the input/output control unit 307 detects the short press and the long press of the upper button 105, the short press and the long press of the middle button 106, and the short press and the long press of the lower button 107, that is, the short press and the long press of all the buttons.

When the upper button 105 is short pressed, the input/output control unit 307 increments ("+1") a setting value that is currently operated.

When the upper button 105 is long pressed, the input/output control unit 307 continuously increments the setting value that is currently operated.

When the middle button 106 is short pressed, the input/output control unit 307 determines a setting value that is currently searched for or selects the setting value from a plurality of options.

When the middle button 106 is long pressed, the input/output control unit 307 moves to the power saving mode. In other words, the power-off state is achieved.

When the lower button 107 is short pressed, the input/output control unit 307 decrements ("-1") the setting value that is currently operated.

When the lower button 107 is long pressed, the input/output control unit 307 continuously decrements the setting value that is currently operated.

In the present embodiment, the blood glucose meter 101 is disclosed.

The cap 108 that protects the optical measuring unit 109 is provided and the micro switch 210 that detects the state in which the cap 108 is attached to or removed from the housing 102 is provided. When the input/output control unit 307 detects that the cap 108 is removed from the housing 102, the input/output control unit 307 moves from the power saving mode to the measuring mode. In this manner, the cap is used as a kind of the operation button 104, so that it is possible to provide the blood glucose meter 101 that has a clear and easy to understand operation system for a patient with a minimum number of operation buttons 104.

The operation buttons 104 are realized by known membrane switches and the like. However, it is possible to employ an electrostatic touch panel or a pressure sensitive touch panel which are widely used in smartphones and the like in recent years. In this case, an operation button to start the touch panel from the power saving mode is required.

A postprandial alarm function is provided, which notifies a patient that the postprandial blood glucose level measurement time is reached, to the blood glucose meter 101. When the input/output control unit 307 detects that the current time obtained from the RTC 204 reaches the alarm sounding time stored in the alarm time memory 309, the input/output control unit 307 sounds an alarm (the buzzer 211), sets the postprandial flag in the postprandial flag memory 310 to logical "true" until a predetermined period of time elapses from the alarm sounding time, and records the postprandial flag in the blood glucose level table 306 along with the blood glucose level measured by the blood glucose level measuring unit 301. The postprandial alarm and the postprandial flag are linked with each other in this manner, so that the patient can more reliably perform the postprandial blood glucose level measuring operation.

Three operation buttons 104, which are the upper button 105, the middle button 106, and the lower button 107, are provided to the blood glucose meter 101. Among them, the upper button 105 provides a function to operate the postprandial flag which indicates whether the measured blood glucose level is postprandial or not and which is stored in the blood glucose level table 306 during the measuring mode in which the blood glucose level is measured. On the other hand, during the content mode in which the blood glucose level recorded in the blood glucose level table 306 is referred to, the upper button 105 provides a function to set a postprandial alarm that notifies a patient that the postprandial blood glucose level measurement time is reached. In this way, when the blood glucose level is measured and when the blood glucose level is checked, functions related to a concept of "postprandial" are assigned to one operation button which is the upper button 105, so that it is possible to realize a blood glucose meter which achieves high functionality and simplicity at the same time and whose operation is easy for the patient to learn.

Three operation buttons 104, which are the upper button 105, the middle button 106, and the lower button 107, are provided to the blood glucose meter 101. During the content mode in which the blood glucose level recorded in the blood glucose level table 306 is referred to, when the lower button 107 is continuously pressed, a starting record of the blood glucose level table 306 is reached. When the lower button 107 is further pressed, an average value of morning blood glucose levels in the last one month which are stored in the blood glucose level table 306 is displayed. In other words, it is possible to seamlessly switch between a blood glucose level display function and an average value display function by only moving a reference record. Therefore, a complicated operation such as mode switching is not required, so that the patient can easily learn the operation.

The five-level evaluation is performed by comparing the blood glucose level measured by the blood glucose level measuring unit 301 of the blood glucose meter 101 with a plurality of threshold values. Five colors are assigned according to the result of the five-level evaluation, and the color of the blood glucose level is displayed on the liquid crystal display 103. Further, a five-color scale is displayed at one end of the liquid crystal display 103, and a color in the scale matched to the background color is integrated with the background color and displayed in a tab shape. The tab-shaped display is performed in this manner, so that it is possible to know how high the value of the measured blood glucose level at first glance by seeing the position of the tab.

The input/output control unit 307 controls a display state of the liquid crystal display 103 according to the color setting and the face display setting stored in the various setting information memory 311. When the color setting is set, the input/output control unit 307 performs the five-level evaluation by comparing the blood glucose level measured by the blood glucose level measuring unit 301 with a plurality of threshold values, assigns five colors according to the evaluation result, and displays the color of the blood glucose level on the liquid crystal display 103. When the setting is to display a face mark, the input/output control unit 307 displays a face mark according to the result of the five-level evaluation on the liquid crystal display 103. In this manner, a detailed setting function related to display is provided to the blood glucose meter 101, so that it is possible to achieve an optimal blood glucose level display function according to patient' s preference and a patient' s visual impairment.

Three operation buttons 104, which are the upper button 105, the middle button 106, and the lower button 107, are provided to the blood glucose meter 101. During the measuring mode in which the blood glucose level is measured, the upper button 105 is assigned with a function to operate the postprandial flag which indicates whether the measured blood glucose level is postprandial or not and which is stored in the blood glucose level table 306. On the other hand, during the content mode in which the blood glucose level recorded in the blood glucose level table 306 is referred to, the upper button 105 is assigned with a function to set a postprandial alarm that notifies a patient that the postprandial blood glucose level measurement time is reached. In any of the measuring mode and the content mode, the middle button 106 is assigned with a function to turn off power. In this manner, different functions are assigned to a small number of operation buttons according to each operation mode, so that it is possible to realize many functions with a minimum number of operation buttons.

While the embodiment of the present invention has been described, the present invention is not limited to the embodiment described above, and the present invention includes other modified examples and application examples without departing from the scope of the present invention described in the claims.

### Reference Signs List

101 blood glucose meter, 102 housing, 103 liquid crystal display, 104 operation button, 105 upper button, 106 middle button, 107 lower button, 108 cap, 109, optical measuring unit, 201 CPU, 202 RAM, 203 EEPROM, 204 RTC, 205 display unit, 206 operation unit, 207 bus, 208 D/A converter, 209 A/D converter, 210 micro switch, 211 buzzer, 212 power supply control unit, 213 driver, 214 LED, 215 photodiode, 216 battery, 301 blood glucose level measuring unit, 302 blood glucose level calculation unit, 303 measurement timer, 304 blood, 305 test paper, 306 blood glucose level table, 307 input/output control unit, 308 operation timer, 309 alarm time memory, 310 postprandial flag memory, 311 setting information memory, 401 pointer

## Claims

1. A blood glucose meter comprising:
a measuring unit to which a measurement test paper for measuring a blood glucose level of a patient is attached;
a cap that covers the measuring unit;
a housing which has the measuring unit and to which the cap is attachably and detachably attached;
a switch that detects that the cap is attached to the housing;
a blood glucose level measuring unit that receives a predetermined signal from the measuring unit and measures the blood glucose level of the patient;
a display unit which is provided in the housing and displays the blood glucose level;
a power supply control unit that controls supply of power to the display unit and the blood glucose level measuring unit; and
an input/output control unit which, when the switch detects that the cap is removed from the housing, moves to a mode, in which the blood glucose level is measured, by activating the display unit and the blood glucose level measuring unit through the power supply control unit and outputs the measured blood glucose level to the display unit.

2. The blood glucose meter according to claim 1, wherein
when the switch detects that the cap is attached to the housing, the input/output control unit stops operations of the display unit and the blood glucose level measuring unit through the power supply control unit.

3. The blood glucose meter according to claim 2, further comprising:
a button which is provided adjacent to the display unit of the housing and from which predetermined operation information is inputted; and
a blood glucose level table in which the blood glucose level is stored by the input/output control unit,
wherein when the input/output control unit detects that the button is pressed in a state in which the cap is not removed from the housing, the input/output control unit moves to a state in which measurement-related information including the blood glucose level stored in the blood glucose level table can be displayed on the display unit or moves to a setting mode in which various settings of the blood glucose meter are performed.

4. The blood glucose meter according to claim 3, wherein
when the input/output control unit detects that the button is pressed in a state in which the cap is removed from the housing, the input/output control unit moves to a mode in which the blood glucose level is measured and outputs the measured blood glucose level to the display unit.

5. The blood glucose meter according to claim 4, wherein
when the switch detects that the cap is removed from the housing in a state in which the blood glucose level stored in the blood glucose level table can be displayed on the display unit in a state in which the cap is not removed from the housing, the input/output control unit moves to the mode in which the blood glucose level is measured and outputs the measured blood glucose level to the display unit.
